(19) Europäisches Patentamt
European Patent Office
Office européen des brevets

(11) Veröffentlichungsnummer: **0 490 233 A1**

(12) # EUROPÄISCHE PATENTANMELDUNG

(21) Anmeldenummer: **91120740.5**

(22) Anmeldetag: **04.03.87**

(51) Int. Cl.⁵: **C12P 21/08**, C12N 5/20, C07K 3/18, C07K 15/26, C07K 17/00, //C12N15/62, C12N15/20

Diese Anmeldung is am 03 - 12 - 1991 als Teilanmeldung zu der unter INID-Kode 60 erwähnten Anmeldung eingereicht worden.

(30) Priorität: **10.03.86 DE 3607835**

(43) Veröffentlichungstag der Anmeldung: **17.06.92 Patentblatt 92/25**

(60) Veröffentlichungsnummer der früheren Anmeldung nach Art. 76 EPÜ: **0 236 920**

(84) Benannte Vertragsstaaten: **AT BE CH DE ES FR GB GR IT LI LU NL SE**

(71) Anmelder: **BOEHRINGER INGELHEIM INTERNATIONAL GmbH**
**Postfach 20**
**W-6507 Ingelheim am Rhein(DE)**

(72) Erfinder: **Hauptmann, Rudolf, Dr.**
**Döllachstrasse 22**
**A-2483 Ebreichsdorf(AT)**
Erfinder: **Swetly, Peter, Prof. Dr.**
**Hietzinger Hauptstrasse 40B**
**A-1130 Wien(AT)**
Erfinder: **Meindl, Peter, Dr.**
**Hockegasse 63/1**
**A-1180 Wien(AT)**
Erfinder: **Adolf, Günther, Mag. Dr.**
**Johannagasse 20/7**
**A-1050 Wien(AT)**
Erfinder: **Falkner, Edgar, Dr.**
**Feldgasse 98**
**A-3420 Kritzendorf(AT)**
Erfinder: **Bodo, Gerhard, Prof. Dr.**
**Belghofergasse 27/5**
**A-1120 Wien(AT)**
Erfinder: **Maurer-Fogy, Ingrid, Dr.**
**Lindauergasse 35**
**A-1238 Wien(AT)**

(54) **Monoclonale Antikörper gegen BglII-Hybridinterferone, deren Verwendung und Verfahren zu ihrer Herstellung.**

(57) Gagenstand der Erfindung sind monoklonale Antikörper die die Aktivität von Bgl II-Hybridinterferonen, bestehend aus einem Teil eines α-Interferons und einem Teil eines omega-Interferons, und die Aktivität von Humaninterferonen des Typs α 2 und omega 1 ganz oder teilweize neutralisieren, die sie sezernierende Hybridzellinien und deren Verwendung zur Reinigung von einem Hybridinterferon, einem Interferon α 2 oder einem Interferon-omega 1.

EP 0 490 233 A1

In Nucleic Acids Res. 13, 4739-4749 (1985) (siehe auch EP-A-0.170.204) wird eine neue Klasse von Typ I Interferonen beschrieben, welche als omega-Interferone bezeichnet werden, die für sie kodierende DNA-Sequenzen, Plasmide, die diese DNA-Sequenzen enthalten, und die neuen Interferone produzierende Organismen.

Bei der Herstellung von größeren Versuchsmengen der neuen omega-Interferone mittels den in den oben erwähnten Publikationen beschriebenen Expressionsplasmiden, z.B. mit pRHW11 oder pRHW12 jeweils transformiert in E. coli HBl0l, zeigte es sich jedoch, daß es wünschenswert wäre, die Expression der neuen omega-Interferone zu steigern und gleichzeitig die erforderliche nachfolgende Reinigung der exprimierten omega-Interferone zu verbessern.

Das Ziel der vorliegenden Erfindung ist, die Reinigung der omega-Interferone zu verbessern, insbesondere da es bisher nicht gelang, eine entsprechende Anti-omega-Interferon-Antikörper-Affinitätssäule nach bekannten Methoden herzustellen.

Überraschenderweise wurde nun gefunden, daß die Verbesserung der Reinigung der neuen omega-Interferone mit Hilfe von neuen monoclonalen Antikörpern, die die Aktivität von BglII-Hybridinterferonen der allgemeinen Formel

$$R_1 - \text{Gln Ile Phe} - R_2 \qquad \text{(I)}$$

CAG ATC TTC
BglII

in der

BglII die gemeinsame BglII-Restriktionsstelle der $\alpha$1-, $\alpha$2-und omega-Interferone,

$R_1$ die Peptidsequenz eines $\alpha$1- oder $\alpha$2-Interferons, die durch die DNA-Sequenz dieser Interferone vor der BglII-Schnittstelle kodiert wird, und $R_2$ die Peptidsequenz eines omega-Interferons, die durch die DNA-Sequenz dieses Interferons nach der BglII-Schnittstelle kodiert wird, oder

$R_1$ die Peptidsequenz eines omega-Interferons, die durch die DNA-Sequenz dieses Interferons vor der BglII-Schnittstelle kodiert wird, und $R_2$ die Peptidsequenz eines $\alpha$1- oder $\alpha$2-Interferons, die durch die DNA-Sequenz dieser Interferone nach der BglII-Schnittstelle kodiert wird, bedeuten, deren N-terminale Met- oder N-Formyl-Met-Derivate und, falls die Peptidsequenz des Hybridinterferons eine Glykosylierungsstelle enthält, deren N-glykosylierte Derivate, und die Aktivität von Humaninterferonen des Typs $\alpha$2 und omegal ganz oder teilweise neutralisieren, gelingt.

Bevorzugte BglII-Hybridinterferone sind in diesem Zusammenhang Interferone bestehend aus IFN-$\alpha$2-(Arg) und IFN-omegal(Glu) oder IFN-omegal(Gly), deren N-terminale Met- oder N-Formyl-Met-Derivate und deren N-glykosylierte Derivate,
besonders bevorzugt sind jedoch Hybridinterferone der Formel

```
                    5                   10                  15
Cys Asp Leu Pro Gln Thr His Ser Leu Gly Ser Arg Arg Thr Leu

                    20                  25                  30
Met Leu Leu Ala Gln Met Arg Arg Ile Ser Leu Phe Ser Cys Leu

                    35                  40                  45
Lys Asp Arg Arg Asp Phe Gly Phe Pro Gln Glu Glu Phe Gly Asn

                    50                  55                  60
Gln Phe Gln Lys Ala Glu Thr Ile Pro Val Leu His Glu Met Ile

                    65                  70                  75
Gln Gln Ile Phe Ser Leu Phe His Thr Glu Arg Ser Ser Ala Ala
```

```
                    80                      85                      90
Trp Asn Met Thr Leu Leu Asp Gln Leu His Thr Gly Leu His Gln

                    95                     100                     105
Gln Leu Gln His Leu Glu Thr Cys Leu Leu Gln Val Val Gly Glu

                   110                     115                     120
Gly Glu Ser Ala -X- Ala Ile Ser Ser Pro Ala Leu Thr Leu Arg

                   125                     130                     135
Arg Tyr Phe Gln Gly Ile Arg Val Tyr Leu Lys Glu Lys Lys Tyr

                   140                     145                     150
Ser Asp Cys Ala Trp Glu Val Val Arg Met Glu Ile Met Lys Ser

                   155                     160                     165
Leu Phe Leu Ser Thr Asn Met Gln Glu Arg Leu Arg Ser Lys Asp

                   170
Arg Asp Leu Gly Ser Ser
```

in der
X für Gly oder Glu steht, deren N-terminale Met- oder N-Formyl-Met-Derivate und deren N-glykosylierte Derivate,
insbesondere jedoch IFN-omegaI/α2(BglII) der Formel

```
                     5                      10                      15
Cys Asp Leu Pro Gln Asn His Gly Leu Leu Ser Arg Asn Thr Leu

                    20                      25                      30
Val Leu Leu His Gln Met Arg Arg Ile Ser Pro Phe Leu Cys Leu

                    35                      40                      45
Lys Asp Arg Arg Asp Phe Arg Phe Pro Gln Glu Met Val Lys Gly

                    50                      55                      60
Ser Gln Leu Gln Lys Ala His Val Met Ser Val Leu His Glu Met

                    65                      70                      75
Leu Gln Gln Ile Phe Asn Leu Phe Ser Thr Lys Asp Ser Ser Ala

                    80                      85                      90
Ala Trp Asp Glu Thr Leu Leu Asp Lys Phe Tyr Thr Glu Leu Tyr

                    95                     100                     105
Gln Gln Leu Asn Asp Leu Glu Ala Cys Val Ile Gln Gly Val Gly

                   110                     115                     120
Val Thr Glu Thr Pro Leu Met Lys Glu Asp Ser Ile Leu Ala Val
```

| | | | | | | | | | | | | | | |
|---|---|---|---|---|---|---|---|---|---|---|---|---|---|---|
| | | | | 125 | | | | | 130 | | | | | 135 |
| Arg | Lys | Tyr | Phe | Gln | Arg | Ile | Thr | Leu | Tyr | Leu | Lys | Glu | Lys | Lys |
| | | | | 140 | | | | | 145 | | | | | 150 |
| Tyr | Ser | Pro | Cys | Ala | Trp | Glu | Val | Val | Arg | Ala | Glu | Ile | Met | Arg |
| | | | | 155 | | | | | 160 | | | | | 165 |
| Ser | Phe | Ser | Leu | Ser | Thr | Asn | Leu | Gln | Glu | Ser | Leu | Arg | Ser | Lys |
| | | | | 170 | | | | | | | | | | |
| Glu | | | | | | | | | | | | | | |

, sowie dessen N-terminales Met- oder N-Formyl-Met-Derivat.

Die neuen Antikörper gegen diese Hybridinterferone, insbesondere gegen das IFN-omegal/α2(BglII), die zugleich auch die parenteralen Interferone IFN-α2 und IFN-omegal erkennen, eignen sich für die Immunaffinitätschromatographie von omega-Interferonen.

Die neuen monoklonalen Antikörper sind daher insbesondere zur Hochreinigung und zum Nachweis von omegal(Glu)- oder omegal(Gly)-Interferon geeignet.

Ein weiterer Gegenstand der vorliegenden Anmeldung sind somit neue monoklonale Antikörper-produzierende Hybridzellinien, Verfahren zu deren Herstellung und ein Verfahren zur Herstellung von monoklonalen Antikörpern der Maus mit Spezifität für IFN-α und omega-Interferon, insbesondere für omegal-Interferon.

Die hierfür erforderlichen Antikörper-produzierenden Hybridzellinien erhält man durch Zellfusion von Milzzellen von mit einem Hybridinterferon wie IFN-omegal/α2(BglII) immunisierten Mäusen mit Myelomzellen, z.B. Myelomzellen der Linie P3-X-63Ag8-653 (siehe Nature 266, 550-552 (1977)). Eine so erhaltene Zellinie sezerniert große Mengen eines Antikörpers, der die antivirale Aktivität sowohl des zur Immunisierung eingesetzten Hybridinterferons als auch der beiden parentalen Interferone, z.B. im Falle des IFN-omegal/α2(BglII) die des omegal-Interferon und des IFN-α2(Arg), neutralisieren kann und für die Affinitätschromatographie von omegal-Interferon geeignet ist.

Der so hergestellte Antikörper kann nach kovalenter Bindung an einen biologisch inaktiven Träger zur Hochreinigung von omegal-Interferone oder IFN-α2 verwendet werden.

Die kovalente Bindung des Antikörpers erfolgt hierbei an einen entsprechend aktivierten Träger, vorzugsweise auf Dextran-Basis, z.B. an CNBr-aktivierte Sepharose oder CH-aktivierte Sepharose der Firma Pharmacia, Uppsala. Zur Hochreinigung wird eine Lösung des zu reinigenden omegal-Interferons, welches zweckmäßigerweise entweder gemäß den in der EP-A-0.170.204 beschriebenen Verfahren oder erfindungsgemäß mit Hilfe der vorstehend beschriebenen neuen Plasmide erhalten wird, bei schwach basischen pH, z.B. bei einem pH 7-8, vorzugsweise jedoch bei einem pH 7,5, über einen so hergestellten Antikörper-Affinitätsträger gepumpt, solange bei pH 7,5 gewaschen bis das Eluat proteinfrei ist, und anschließend das gebundene Interferon im sauren Bereich, z.B. mit Hilfe von 0,1 molarer Zitronensäure in 25%igem Äthylenglykol, eluiert. Die so erhaltenen proteinhältigen Fraktionen werden anschließend über einen stark sauren Kationenaustauscher, z.B. den Kationenaustauscher Mono-S der Firma Pharmacia, chromatographiert. Hierbei wird das Humaninterferon des obigen Eluats sofort von der Kationenaustauscher-Säule adsorbiert und anschließend mit Hilfe eines NaCl-Gradienten eluiert.

Die zur Herstellung der neuen monoclonalen Antikörper der vorliegenden Erfindung verwendeten Hybridinterferone sind aus einem Teil eines α-Interferons, vorzugsweise aus einem Teil eines α1- oder α2-Interferons, z.B. des IFN-α2(Arg) (siehe EP-A-0.095.702), und einem Teil eines omega-Interferons, vorzugsweise aus einem Teil des omegal(Glu)- oder omegal(Gly)-Interferons (siehe EP-A-0.170.204), aufgebaut. Hierbei erfolgt beispielsweise beim IFN-α2(Arg) und IFN-omegal die Verknüpfung der für die entsprechenden Teile kodierende DNA-Sequenzen über die BglII-Schnittstelle, die sich in den Positionen 191-196 in beiden Genen befindet. Hierbei muß eine gegebenenfalls vorhandende Lücke in das Peptid von Aminosäure 1 bis 66 eines α-Interferons, z.B. die Lücke für die Aminosäure Nr. 45 im IFN-α2(Arg), mitgezählt werden, damit die Sequenz beider Gene miteinander verglichen werden kann, wie aus der nachfolgenden Abbildung hervorgeht (einschließlich des N-terminalen Met-Restes, welcher nach der bakteriellen Proteinsynthese meistens wieder abgespalten wird):

```
                    5                   10                  15
Met Cys Asp Leu Pro Gln Thr His Ser Leu Gly Ser Arg Arg Thr
ATG TGT GAT CTG CCT CAA ACC CAC AGC CTG GGT AGC AGG AGG ACC  45
Met Cys Asp Leu Pro Gln Asn His Gly Leu Leu Ser Arg Asn Thr
ATG TGT GAT CTG CCT CAG AAC CAT GGC CTA CTT AGC AGG AAC ACC  45

                    20                  15                  30
Leu Met Leu Leu Ala Gln Met Arg Arg Ile Ser Leu Phe Ser Cys
TTG ATG CTC CTG GCA CAG ATG AGG AGA ATC TCT CTT TTC TCC TGC  90
Leu Val Leu Leu His Gln Met Arg Arg Ile Ser Pro Phe Leu Cys
TTG GTG CTT CTG CAC CAA ATG AGG AGA ATC TCC CCT TTC TTG TGT  90

                    35                  40                  45
Leu Lys Asp Arg Arg Asp Phe Gly Phe Pro Gln Glu Glu Phe ...
TTG AAG GAC AGA CGT GAC TTT GGA TTT CCC CAG GAG GAG TTT ... 135
Leu Lys Asp Arg Arg Asp Phe Arg Phe Pro Gln Glu Met Val Lys
CTC AAG GAC AGA AGA GAC TTC AGG TTC CCC CAG GAG ATG GTA AAA 135

                    50                  55                  60
Gly Asn Gln Phe Gln Lys Ala Glu Thr Ile Pro Val Leu His Glu
GGC AAC CAG TTC CAA AAG GCT GAA ACC ATC CCT GTC CTC CAT GAG 180
Gly Ser Gln Leu Gln Lys Ala His Val Met Ser Val Leu His Glu
GGG AGC CAG TTG CAG AAG GCC CAT GTC ATG TCT GTC CTC CAT GAG 180
```

```
                    65                       70                       75
Met Ile Gln Gln Ile Phe Asn Leu Phe Ser Thr Lys Asp Ser Ser
ATG ATC CAG CAG ATC TTC AAT CTC TTC AGC ACA AAG GAC TCA TCT 225
Met Leu Gln Gln Ile Phe Ser Leu Phe His Thr Glu Arg Ser Ser
ATG CTG CAG CAG ATC TTC AGC CTC TTC CAC ACA GAG CGC TCC TCT 225

                    80                       85                       90
Ala Ala Trp Asp Glu Thr Leu Leu Asp Lys Phe Tyr Thr Glu Leu
GCT GCT TGG GAT GAG ACC CTC CTA GAC AAA TTC TAC ACT GAA CTC 270
Ala Ala Trp Asn Met Thr Leu Leu Asp Gln Leu His Thr Gly Leu
GCT GCC TGG AAC ATG ACC CTC CTA GAC CAA CTC CAC ACT GGA CTT 270

                    95                      100                      105
Tyr Gln Gln Leu Asn Asp Leu Glu Ala Cys Val Ile Gln Gly Val
TAC CAG CAG CTG AAT GAC CTG GAA GCC TGT GTG ATA CAG GGG GTG 315
His Gln Gln Leu Gln His Leu Glu Thr Cys Leu Leu Gln Val Val
CAT CAG CAA CTG CAA CAC CTG GAG ACC TGC TTG CTG CAG GTA GTG 315

                   110                      115                      120
Gly Val Thr Glu Thr Pro Leu Met Lys Glu Asp Ser Ile Leu Ala
GGG GTG ACA GAG ACT CCC CTG ATG AAG GAG GAC TCC ATT CTG GCT 360
Gly Glu Gly Glu Ser Ala Gly Ala Ile Ser Ser Pro Ala Leu Thr
GGA GAA GGA GAA TCT GCT GGG GCA ATT AGC AGC CCT GCA CTG ACC 360

                   125                      130                      135
Val Arg Lys Tyr Phe Gln Arg Ile Thr Leu Tyr Leu Lys Glu Lys
GTG AGG AAA TAC TTC CAA AGA ATC ACT CTC TAT CTG AAA GAG AAG 405
Leu Arg Arg Tyr Phe Gln Gly Ile Arg Val Tyr Leu Lys Glu Lys
TTG AGG AGG TAC TTC CAG GGA ATC CGT GTC TAC CTG AAA GAG AAG 405

                   140                      145                      150
Lys Tyr Ser Pro Cys Ala Trp Glu Val Val Arg Ala Glu Ile Met
AAA TAC AGC CCT TGT GCC TGG GAG GTT GTC AGA GCA GAA ATC ATG 450
Lys Tyr Ser Asp Cys Ala Trp Glu Val Val Arg Met Glu Ile Met
AAA TAC AGC GAC TGT GCC TGG GAA GTT GTC AGA ATG GAA ATC ATG 450
```

```
                    155                 160                 165
Arg Ser Phe Ser Leu Ser Thr Asn Leu Gln Glu Ser Leu Arg Ser
AGA TCT TTT TCT TTG TCA ACA AAC TTG CAA GAA AGT TTA AGA AGT 495
Lys Ser Leu Phe Leu Ser Thr Asn Met Gln Glu Arg Leu Arg Ser
AAA TCC TTG TTC TTA TCA ACA AAC ATG CAA GAA AGA CTG AGA AGT 495


                    170
Lys Glu
AAG GAA TGA                                                 504
Lys Asp Arg Asp Leu Gly Ser Ser
AAA GAT AGA GAC CTG GGC TCA TCT TGA                         522
```

In der obigen Abbildung stellt in jeder Doppelreihe die erste Reihe die entsprechenden Sequenzen des IFN-$\alpha$2(Arg) und die zweite Reihe die des omegal-Interferons dar, hierbei befindet sich in den Nukleotidpositionen 191-196 die gemeinsame BglII-Schnittstelle für beide Gene - das IFN-$\alpha$2(Arg)-Gen weist an der Nukleotidposition 451-456 eine zweite BglII-Schnittstelle auf.

Ferner enthalten die nachfolgenden nicht maßstabgerechten Darstellungen der einzelnen Plasmide nur die wesentlichen Sequenzen und Restriktionsenzymerkennungsstellen. Hierbei wurden folgende Abkürzungen benutzt:

Restriktionsenzymerkennungssequenzen:
B: BamHI, Bg: BglII, Bg1: 1.BglII-Stelle im IFN-$\alpha$2(Arg)-Gen,
Bg2: 2.BglII-Stelle im IFN-$\alpha$2(Arg)-Gen,
E: EcoRI, H: HindIII, N: NcoI, P: PstI, S: SphI
p/o: Tryptophan Promotor/Operator (Serratia marcescens) mit anschließender Shine-Dalgarno Sequenz (ribosomale Bindungsstelle)
par: partition Lokus aus dem Plasmid pPM31
ori: Replikationsursprung
$Ap^r$: Ampicillin Resistenzgen
$Tc^r$: Tetracyclin Resistenzgen
$Tc^s$: Tetracyclin sensitiv
kb: 1000 Basenpaare

Desweiteren sind in der vorliegenden Anmeldung folgende Figuren enthalten:

- Fig. 1: Konstruktionsschema für parpATER33
- Fig. 2: Konstruktionsschema für pRHW14
- Fig. 3: Autoradiogramm von $^{35}S$ markierten Proteinen aus Maxizellen (E.coli CSR 603)
- Fig. 4: Konstruktionsschema für pRH72
- Fig. 5: Konstruktionsschema für pRH78r und pRH78f
- Fig. 6: MONO-S Chromatogramm von IFN-omegal/$\alpha$2(BglII)
  AUFS = Absorption units full scale
- Fig. 7: Gelpermeations-HPLC von IFN-omegal/$\alpha$2(BglII)
- Fig. 8: MONO-S Chromatogramm von IFN-omegal
- Fig. 9: Reverse Phase-HPLC von IFN-omegal

Die neuen BglII-Hybridinterferone und deren N-glykosylierte Derivate bestehen also entweder aus den Aminosäuren 1-66 eines $\alpha$1- oder $\alpha$2-Interferons, vorzugsweise aus den Aminosäuren 1-65 des IFN-$\alpha$2(Arg), und den Aminosäuren 67 bis 173 eines omegal-Interferons oder aus den Aminosäuren 1 bis 66 des omegal-Interferons und den Aminosäuren 67 bis 167 eines $\alpha$1- oder $\alpha$2-Interferons, vorzugsweise aus den Aminosäuren 66 bis 166 des IFN-$\alpha$2(Arg), wobei der N-terminale Met-Rest nach der bakteriellen Proteinsynthese meistens wieder abgespalten wird.

Überraschenderweise werden die neuen Hybridinterferone von Anti-IFN-$\alpha$-Antikörpern erkannt. Dadurch wird die Reinigung der neuen Hybridinterferone mittels literaturbekannter Anti-IFN-$\alpha$-Antikörpern (siehe beispielsweise EP-A-0.119.476) möglich. Mit einem so erhaltenen reinen neuen Hybridinterferon kann dann durch Immunisierung eines Versuchstieres wie BALB/c-Mäusen die Bildung des entsprechenden Antikörpers induziert werden. Diese neuen Antikörper erkennen nicht nur die neuen Hybridinterferone, sondern

überraschenderweise auch die einzelnen Bausteine der Hybridinterferone, z.B. ein omega-Interferon.

Die Ausgangsbasis zur Konstruktion der entsprechenden Hybridinterferon-Plasmide ist somit die gemeinsame BglII-Restriktionsstelle in den Positionen 191-196 eines α1-, α2- und omegal-Interferongens, wie dies bereits vorstehend erwähnt wurde, wobei die Lücke für das Codon Nr. 45 im IFN-α2(Arg)-Gen mitgezählt wurde, sowie zur Isolierung des entsprechenden, erforderlichen Gens die für ein α1-, α2-Interferon bzw. für ein omegal-Interferon kodierenden Plasmide.

Hierzu wird nun zuerst ein die omegal-Interferon-Expression verbesserndes neues Plasmid hergestellt. Hierbei dient als Ausgangsplasmid das literaturbekannte und käuflich erhältliche Plasmid pAT153 (Fa. Amersham, siehe auch A.J. Twigg et al. in Nature 283, 216-218 (1980)), beispielsweise das für IFN-α2(Arg) kodierende Plasmid parpER33 (siehe EP-A-0.115.613) und ein für ein omegal-Interferon der Formel

```
                    5                   10                  15
Cys Asp Leu Pro Gln Asn His Gly Leu Leu Ser Arg Asn Thr Leu

                    20                  25                  30
Val Leu Leu His Gln Met Arg Arg Ile Ser Pro Phe Leu Cys Leu

                    35                  40                  45
Lys Asp Arg Arg Asp Phe Arg Phe Pro Gln Glu Met Val Lys Gly

                    50                  55                  60
Ser Gln Leu Gln Lys Ala His Val Met Ser Val Leu His Glu Met

                    65                  70                  75
Leu Gln Gln Ile Phe Ser Leu Phe His Thr Glu Arg Ser Ser Ala

                    80                  85                  90
Ala Trp Asn Met Thr Leu Leu Asp Gln Leu His Thr Gly Leu His

                    95                  100                 105
Gln Gln Leu Gln His Leu Glu Thr Cys Leu Leu Gln Val Val Gly

                    110                 115                 120
Glu Gly Glu Ser Ala -X- Ala Ile Ser Ser Pro Ala Leu Thr Leu

                    125                 130                 135
Arg Arg Tyr Phe Gln Gly Ile Arg Val Tyr Leu Lys Glu Lys Lys

                    140                 145                 150
Tyr Ser Asp Cys Ala Trp Glu Val Val Arg Met Glu Ile Met Lys

                    155                 160                 165
Ser Leu Phe Leu Ser Thr Asn Met Gln Glu Arg Leu Arg Ser Lys

                    170
Asp Arg Asp Leu Gly Ser Ser
```

in der

X in Position III für Glu oder Gly steht,

kodierendes Plasmid wie das Plasmid pRHW11 oder 12 (siehe EP-A-0.170.204):

Das Plasmid parpER33, welches den par-Lokus mit der Sequenz der Formel

```
GAATTCCGAC AGTAAGACGG GTAAGCCTGT TGATGATACC GCTGCCTTAC   50
TGGGTGCATT AGCCAGTCTG AATGACCTGT CACGGGATAA TCCGAAGTGG  100
TCAGACTGGA AAATCAGAGG GCAGGAACTG CTGAACAGCA AAAAGTCAGA  150
TAGCACCACA TAGCAGACCC GCCATAAAAC GCCCTGAGAA ZCCGTGACGG  200

GCTTTTCTTG TATTATGGGT AGTTTCCTTG CATGAATCCA TAAAAGGCGC  250
CTGTAGTGCC ATTTACCCCC ATTCACTGCC AGAGCCGTGA GCGCAGCGAA  300
CTGAATGTCA CGAAAAAGAC AWCGACTCAG GTGCCTGATG GTCGGAGACA  350
AAAGGAATAT TCAGCGATTT GCCCGAGGAA TTC                    383
```

in der

Z das Nukleotid G oder C und

W das Nukleotid G oder kein Nukleotid bedeuten, sowie die für die Expression erforderlichen Replikon- und Kontrollsequenzen des Plasmids pER103 (siehe Anspruch 17) enthält, wird mit BamHI und PstI geschnitten. Die dabei entstehenden beiden Fragmente wurden mittels Gelelektrophorese, z.B. mit 1%igem Agarosegel, aufgetrennt und das kleinere Fragment, welches das IFN-$\alpha$2(Arg)-Gen enthält, wurde mittels Elektroelution isoliert. Die so erhaltene DNA wurde anschließend durch Zusatz von Äthanol ausgefällt, abzentrifugiert und in einem geeigneten Puffer wie TE-Puffer, z.B. in 10 mMol Tris(hydroxymethyl)aminomethan(Tris), pH = 8,0, und 1 mMol Äthylendinitrilotetraessigsäure-dinatriumsalz (EDTA)) aufgenommen.

Das Plasmid pAT153 wird ebenfalls mit BamHI und PstI geschnitten. Die dabei entstehenden beiden Fragmente wurden mittels Gelelektrophorese, z.B. mit 1%igem Agarosegel, aufgetrennt, und das größere Fragment, welches den Replikationsursprung enthält, isoliert. Das so erhaltene größere Fragment wird anschließend mit dem aus dem Plasmid parpER33 erhaltenen kleineren Fragment in Gegenwart einer Ligase, z.B. $T_4$ DNA-Ligase, ligiert. Zur Replikation der entstehenden Plasmide werden Bakterien, vorzugsweise E.coli vom Stamm HBl0l (Genotyp $F^-$, hsdS20($r^-$, $m^-$), recA13,
ara-14, proA2, lacY1, galK2, rpsL20 ($Sm^r$), xyl-5, mtl-l, supE44, $lambda^-$), mit einer $CaCl_2$-Lösung gewaschen, und die so erhaltenen, kompetenten E.coli HB l0l mit der Ligasereaktionsmischung vermischt, und nach Inkubation bei 0°C wird die Plasmid-DNA durch Hitzeschock bei 42°C für 2 Minuten von den Bakterien aufgenommen. Anschließend werden die transformierten Bakterien auf Ampicillin-haltigen LB-Agar (LB-Medium + 15 g/l Agar) plattiert. Auf diesem Agar können nur E. coli HBl0l-Bakterien wachsen, die ein rekombinantes Vektormolekül aufgenommen haben. Nach Inkubation bei 37°C wurden 12 der entstandenen Kolonien ausgewählt und von diesen nach der Methode von Birnboim et al. (siehe Nucl. Acids Res. 7, 1513 (1979)) die Plasmide isoliert. Durch Restriktionsenzymdoppelverdauung der ausgewählten Plasmide mit PstI-BamHI, PstI-PvuII oder EcoRI-BamHI und anschließender Gelelektrophorese der erhaltenen Fragmente wurde die Richtigkeit der Konstruktion dieser Plasmide bestätigt. Ein so erhaltenes Plasmid wurde ausgewählt und mit parpATER33 (Konstruktionsschema: siehe Figur 1) bezeichnet. Dieses Plasmid zeigt transformiert in E. coli HBl0l den Phänotyp $Ap^r$ (Ampicillin-Resistenz), $Tc^r$ (Tetracyclin-Resistenz).

Das so erhaltene Plasmid parpATER33 mit der Restriktionskarte

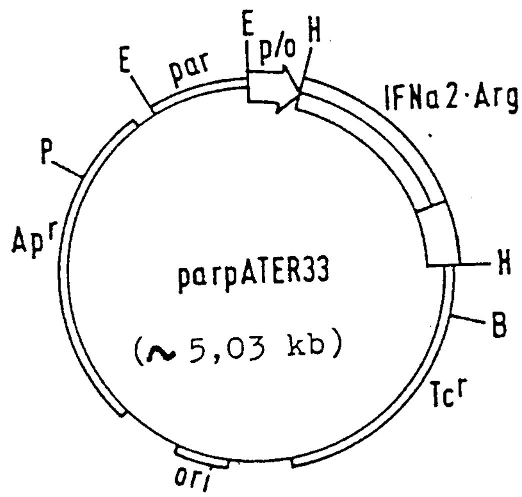


wird zur Herstellung eines die Expression von omegal-Interferon verbessernden Plasmids (Konstruktionsschema: siehe Figur 2) mit HindIII und BamHI geschnitten. Die dabei entstandenden drei Fragmente werden mittels Gelelektrophorese, z.B. mit 1%igem Agarosegel, aufgetrennt und das größte, ca. 3750 Basenpaare (bp) lange Fragment (Fragment a), welches den Tryptophan-Promotor/Operator (Serratia marcescens), den Replikationsursprung und das $Ap^r$-Gen trägt, isoliert und mit der für ein omegal-Interferon kodierenden DNA ligiert. Diese DNA erhält man durch Schneiden eines für omegal-Interferon kodierenden Plasmid, vorzugsweise durch Schneiden des Plasmids pRHW11 bzw. pRHW12 mit BamHI und HindIII und anschließendem Auftrennen der beiden so erhaltenen Fragmente mittels Gelelektrophorese, wobei das gewünschte Gen in dem kleineren, ca. 800 bp langen Fragment enthalten ist. Zur Replikation der entstandenen Plasmide werden Bakterien, vorzugsweise E. coli HBl0l, mit einer $CaCl_2$-Lösung gewaschen und die so erhaltenen, kompetenten E. coli HBl0l mit der Ligasereaktionsmischung vermischt, und nach Inkubation bei 0 ° C wird die Plasmid-DNA durch Hitzeschock bei 42 ° C für 2 Minuten von den Bakterien aufgenommen. Anschließend werden die transformierten Bakterien auf Ampicillin-haltigen LB-Agar plattiert. Auf diesem Agar können nur E. coli HBl0l-Bakterien wachsen, die ein rekombinantes Vektormolekül aufgenommen haben. Nach Inkubation bei 37 ° C werden 6 der entstandenen Kolonien ausgewählt, und von diesen nach der Methode von Birnboim et al. (siehe Nucl. Acids Res. 7, 1513 (1979)) die Plasmide isoliert. Durch Restriktionsenzymver- dauung der ausgewählten Plasmide mit EcoRI, HindIII, NcoI und PstI und anschließender Gelelektrophorese wurde die Richtigkeit der Konstruktion dieser Plasmide bestätigt. Ein so erhaltenes Plasmid mit der Restriktionskarte

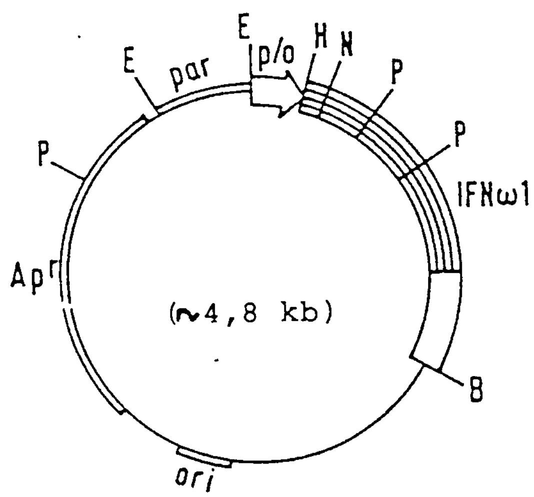


, wobei IFN ω1 die für IFN-omegal(Gly) oder IFN-omegal(Glu) kodierende DNA-Sequenz darstellt, wurde ausgewählt, und mit pRHW14 (siehe Figur 2) bezeichnet, wenn als Ausgangsplasmid das Plasmid pRHW12,

welches für omegal(Gly)-Interferon kodiert, verwendet wird. Dieses Plasmid zeigt transformiert in E. coli HBl0l den Phänotyp $Ap^r$ und $Tc^s$.

Bei Verwendung von pRHW11 als Ausgangsplasmid erhält man analog das Plasmid pRHW13, welches für omegal(Glu)-Interferon kodiert.

Das so erhaltene Plasmid pRHW14 weist eine doppelt so große Expressionsrate für das omegal(Gly)-Interferon auf als das bisher bekannte Plasmid pRHW12 (siehe EP-A-0.170.204) wie im Maxizell-System (siehe J. Bacteriol. 137, 692-693 (1979)) nachgewiesen werden kann. Hierzu wird E. coli CSR603 (Genotyp $F^-$, thr-l, leuB6, proA2, phr-l, recA1, argE3, thi-l, uvrA6, ara-14, lacY1, galK2, xyl-5, mtl-l, gyrA98 (nalA98), rpsL31, tsx-33, $lambda^-$, supE44), welcher defizient im Reparatursystem für UV-induzierte Schäden ist, mit den Plasmiden pRHW12 und pRHW14 transformiert. Wird die Strahlendosis so gewählt, daß zwar das Bakterienchromosom oft, die Plasmide aber kaum geschädigt werden, so erfolgt Transkription und in der Folge Translation vorwiegend nur von Plasmid-kodierten Genen. Enthält das Medium $^{35}$S-Methionin, so werden hauptsächlich Plasmid-Genprodukte markiert, die nach Auftrennen auf einem Acrylamidgel unter Verwendung einer Verstärkerfolie direkt mit Hilfe eines Röntgenfilms registriert werden können (siehe Figur 3). Das Ampicillin-Resistenzgenprodukt ($\beta$-Lactamase, bla) wird in beiden Fällen gleich stark markiert. Im Falle des pRHW14 ist jedoch das omegal(Gly)-Interferon doppelt so stark markiert wie beim pRHW12.

Zur Herstellung eines für ein Hybridinterferon der Formel I kodierenden Plasmids, das vor der gemeinsamen BglII-Schnittstelle die für $\alpha$1- oder $\alpha$2-Interferon kodierende DNA-Sequenzen enthält (Konstruktionsschema: siehe Figur 4), wird beispielsweise das Plasmid parpATER33 und ein für ein omegal-Interferon kodierendes Plasmid wie das Plasmid pRHW14 jeweils mit BglII und SphI geschnitten. Die hierbei entstandenen Fragmente wurden mittels Gelelektrophorese, z.B. mit 1%igem Agarosegel, aufgetrennt, eluiert und mittels Äthanol-Präzipitation gereinigt. Nach Lösen der Fragmente in einem geeigneten Puffer, z.B. in TE-Puffer, wurde das aus dem Plasmid parpATER33 erhaltene, große Fragment mit dem aus dem Plasmid pRHW14 erhaltenen, kleineren Fragment in Gegenwart einer Ligase, z.B. $T_4$ DNA-Ligase, ligiert. Zur Replikation der entstandenen Plasmide wurden Bakterien, vorzugsweise E.coli vom Stamm HBl0l (Genotyp $F^-$, hsdS20($r^-$, $m^-$), recA13, ara-14, proA2, lacY1, galK2, rpsL20 ($Sm^r$), xyl-5, mtl-l, supE44, $lambda^-$), mit einer $CaCl_2$-Lösung gewaschen, und die so erhaltenen kompetenten E.coli HB l0l mit der Ligasereaktionsmischung vermischt, und nach Inkubation bei 0 °C wird die Plasmid-DNA durch Hitzeschock bei 42 °C für 2 Minuten von den Bakterien aufgenommen. Anschließend werden die transformierten Bakterien auf Ampicillin-haltigen LB-Agar (LB-Medium + 15 g/l Agar) plattiert. Auf diesem Agar können nur E. coli HBl0l-Bakterien wachsen, die ein rekombinantes Vektormolekül aufgenommen haben. Nach Inkubation bei 37 °C wurden mehrere der entstandenen Kolonien ausgewählt und von diesen nach der Methode von Birnboim et al. (siehe Nucl. Acids Res. 7, 1513 (1979)) die Plasmide isoliert. Durch Restriktionsenzymdoppelverdauung der ausgewählten Plasmide mit BglII und SphI und anschließender Gelelektrophorese der erhaltenen Fragmente wurde die Richtigkeit der Konstruktion dieser Plasmide bestätigt. Ein so erhaltenes Plasmid wurde ausgewählt und mit pRH72 bezeichnet. Dieses Plasmid mit der Restriktionskarte

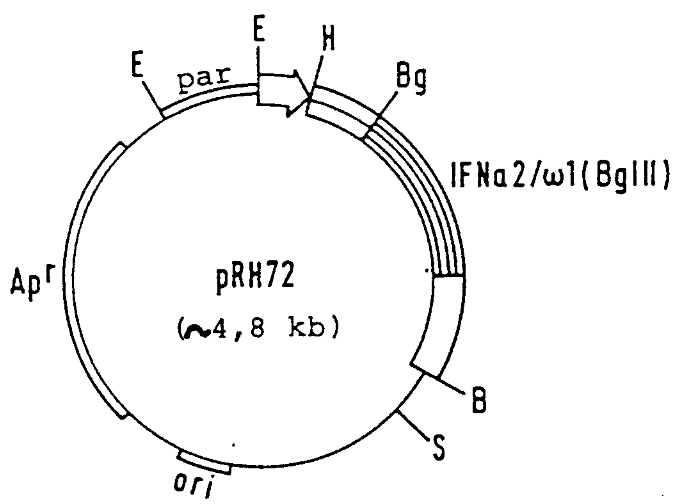


zeigt transformiert in E. coli HBl0l den Phänotyp $Ap^r$ und $Tc^s$ und kodiert für das IFN-$\alpha$2/omegal(Gly)(BglII) der Formel

```
                    5                   10                  15
Cys Asp Leu Pro Gln Thr His Ser Leu Gly Ser Arg Arg Thr Leu

                    20                  25                  30
Met Leu Leu Ala Gln Met Arg Arg Ile Ser Leu Phe Ser Cys Leu

                    35                  40                  45
Lys Asp Arg Arg Asp Phe Gly Phe Pro Gln Glu Glu Phe Gly Asn

                    50                  55                  60
Gln Phe Gln Lys Ala Glu Thr Ile Pro Val Leu His Glu Met Ile

                    65                  70                  75
Gln Gln Ile Phe Ser Leu Phe His Thr Glu Arg Ser Ser Ala Ala

                    80                  85                  90
Trp Asn Met Thr Leu Leu Asp Gln Leu His Thr Gly Leu His Gln

                    95                  100                 105
Gln Leu Gln His Leu Glu Thr Cys Leu Leu Gln Val Val Gly Glu

                    110                 115                 120
Gly Glu Ser Ala Gly Ala Ile Ser Ser Pro Ala Leu Thr Leu Arg

                    125                 130                 135
Arg Tyr Phe Gln Gly Ile Arg Val Tyr Leu Lys Glu Lys Lys Tyr

                    140                 145                 150
Ser Asp Cys Ala Trp Glu Val Val Arg Met Glu Ile Met Lys Ser

                    155                 160                 165
Leu Phe Leu Ser Thr Asn Met Gln Glu Arg Leu Arg Ser Lys Asp

                    170
Arg Asp Leu Gly Ser Ser
```

, dessen Met- und N-Formyl-Met-Derivat sowie dessen N-glykosyliertes Derivat, und enthält die für dieses Peptid kodierende Sequenz der Formel

```
ATG TGT GAT CTG CCT CAA ACC CAC AGC CTG GGT AGC AGG AGG ACC  45
TTG ATG CTC CTG GCA CAG ATG AGG AGA ATC TCT CTT TTC TCC TGC  90
TTG AAG GAC AGA CGT GAC TTT GGA TTT CCC CAG GAG GAG TTT GGC 135
AAC CAG TTC CAA AAG GCT GAA ACC ATC CCT GTC CTC CAT GAG ATG 180
ATC CAG CAG ATC TTC AGC CTC TTC CAC ACA GAG CGC TCC TCT GCT 225
GCC TGG AAC ATG ACC CTC CTA GAC CAA CTC CAC ACT GGA CTT CAT 270
CAG CAA CTG CAA CAC CTG GAG ACC TGC TTG CTG CAG GTA GTG GGA 315
GAA GGA GAA TCT GCT GGG GCA ATT AGC AGC CCT GCA CTG ACC TTG 360
AGG AGG TAC TTC CAG GGA ATC CGT GTC TAC CTG AAA GAG AAG AAA 405
```

```
TAC AGC GAC TGT GCC TGG GAA GTT GTC AGA ATG GAA ATC ATG AAA 450
TCC TTG TTC TTA TCA ACA AAC ATG CAA GAA AGA CTG AGA AGT AAA 495
GAT AGA GAC CTG GGC TCA TCT TGA                             519
```

oder deren degenerierte Varianten.

Zur Herstellung eines für ein Hybridinterferon der Formel I kodierenden Plasmids, das vor der gemeinsamen BglII-Schnittstelle die für ein omegaI-Interferon kodierende DNA-Sequenz enthält (Konstruktionsschema: siehe Figur 5), muß, falls in der DNA-Sequenz des $\alpha$-Interferons zwei BglII-Schnittstellen wie in der DNA-Sequenz des IFN-$\alpha$2(Arg) vorhanden sind, in zwei Schritten verfahren werden: Im ersten Schritt wird das aus dem Plasmid pRHW14 durch Schnitt mit BglII und SphI gewonnene große Fragment mit dem aus dem Plasmid parpATER33 durch Verdauen mit BglII und SphI gewonnene Fragment, welches den C-Terminus des IFN-$\alpha$2(Arg) kodiert, in Gegenwart einer Ligase, z.B. $T_4$ DNA-Ligase, ligiert. Zur Replikation der entstandenen Plasmide wurden Bakterien, vorzugsweise E. coli HB l0l, wie vorstehend bei der Herstellung des Plasmids pRH72 beschrieben, transformiert, kultiviert und auf ihre Konstruktion hin überprüft.

Ein so erhaltenes Plasmid wurde ausgewählt und mit pRH77 bezeichnet, dieses weist folgende Restriktionskarte auf:

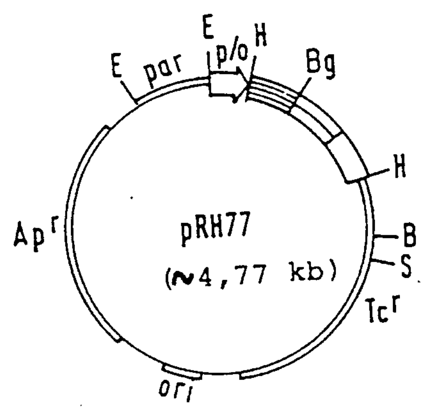


Im 2. Schritt muß nunmehr, um das Gen für das Hybridinterferon, z.B. des IFN-omegaI/$\alpha$2(BglII), zu komplettieren, das beim Schneiden von parpATER33 entfernte Fragment mit den Positionen 192-455 wieder in das Plasmid pRH77 eingebracht werden. Hierzu wird das Plasmid pRH77 mit BglII geschnitten, und das 5'-terminale Phosphat mit Kalbsdarm-Phosphatase (CIP) entfernt. Die so erhaltene linearisierte Form des Plasmids pRHW77 wurde durch Gelelektrophorese, z.B. mit einem 1%igen Agarosegel, aufgetrennt, isoliert und mittels Äthanol-Präzipitation gereinigt. Nach Lösen des so erhaltenen linearisierten Fragments in einem geeigneten Puffer, z.B. in TE-Puffer, wurde dieses mit dem beim ursprünglichen Verdauen von parpATER33 mit BglII und SphI erhaltenen 263 bp langen Fragment in Gegenwart einer Ligase, z.B. $T_4$ DNA-Ligase, ligiert. Zur Replikation der entstandenen Plasmide werden Bakterien, vorzugsweise E. coli HBl0l, wie vorstehend bei der Herstellung des Plasmids pRH72 beschrieben, transformiert, kultiviert und auf ihre Konstruktion hin durch Schnitt mit den Restriktionsendonucleasen AluI oder HaeIII auf ihre Richtigkeit hin untersucht. Die Insertion des 263 pb langen Fragments erfolgt hierbei auf Grund der identischen Enden in zwei Orientierungen.

Das Plasmid, in dem das 263 bp lange BglII-Fragment in der für die Expression richtigen Lage insertiert wurde, wurde mit pRH78r, und jenes mit der für die Expression falschen Orientierung mit pRH78f bezeichnet.

Beide Plasmide zeigen transformiert in E. coli HBl0l den Phänotyp $Ap^r$ und $Tc^r$. Das Plasmid pRH78r mit der Restriktionskarte

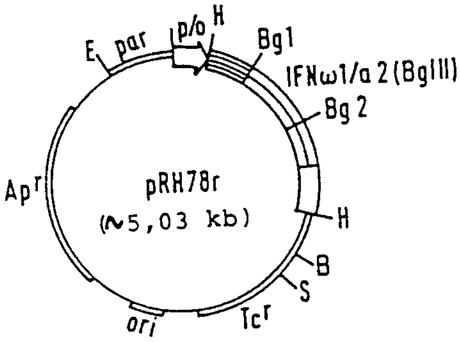


kodiert folgende Polypeptidesequenz für das IFN-omegal/α2(BglII) und enthält die für dieses Peptid kodierende Sequenz:

```
                    5                        10                       15
Met Cys Asp Leu Pro Gln Asn His Gly Leu Leu Ser Arg Asn Thr
ATG TGT GAT CTG CCT CAG AAC CAT GGC CTA CTT AGC AGG AAC ACC  45
                    20                       25                       30
Leu Val Leu Leu His Gln Met Arg Arg Ile Ser Pro Phe Leu Cys
TTG GTG CTT CTG CAC CAA ATG AGG AGA ATC TCC CCT TTC TTG TGT  90
                    35                       40                       45
Leu Lys Asp Arg Arg Asp Phe Arg Phe Pro Gln Glu Met Val Lys
CTC AAG GAC AGA AGA GAC TTC AGG TTC CCC CAG GAG ATG GTA AAA 135
                    50                       55                       60
Gly Ser Gln Leu Gln Lys Ala His Val Met Ser Val Leu His Glu
GGG AGC CAG TTG CAG AAG GCC CAT GTC ATG TCT GTC CTC CAT GAG 180
                    65                       70                       75
Met Leu Gln Gln Ile Phe Asn Leu Phe Ser Thr Lys Asp Ser Ser
ATG CTG CAG CAG ATC TTC AAT CTC TTC AGC ACA AAG GAC TCA TCT 225
                    80                       85                       90
Ala Ala Trp Asp Glu Thr Leu Leu Asp Lys Phe Tyr Thr Glu Leu
GCT GCT TGG GAT GAG ACC CTC CTA GAC AAA TTC TAC ACT GAA CTC 270
                    95                       100                      105
Tyr Gln Gln Leu Asn Asp Leu Glu Ala Cys Val Ile Gln Gly Val
TAC CAG CAG CTG AAT GAC CTG GAA GCC TGT GTG ATA CAG GGG GTG 315
                    110                      115                      120
Gly Val Thr Glu Thr Pro Leu Met Lys Glu Asp Ser Ile Leu Ala
GGG GTG ACA GAG ACT CCC CTG ATG AAG GAG GAC TCC ATT CTG GCT 360
                    125                      130                      135
Val Arg Lys Tyr Phe Gln Arg Ile Thr Leu Tyr Leu Lys Glu Lys
GTG AGG AAA TAC TTC CAA AGA ATC ACT CTC TAT CTG AAA GAG AAG 405
                    140                      145                      150
Lys Tyr Ser Pro Cys Ala Trp Glu Val Val Arg Ala Glu Ile Met
AAA TAC AGC CCT TGT GCC TGG GAG GTT GTC AGA GCA GAA ATC ATG 450

                    155                      160                      165
Arg Ser Phe Ser Leu Ser Thr Asn Leu Gln Glu Ser Leu Arg Ser
AGA TCT TTT TCT TTG TCA ACA AAC TTG CAA GAA AGT TTA AGA AGT 495

Lys Glu
AAG GAA TGA                                                 504
```

Das Plasmid pRH78f mit der Restriktionskarte

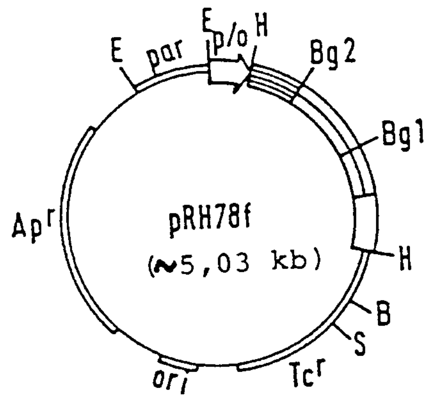


kodiert folgende Polypeptidesequenz und enthält die für dieses Peptid kodierende Sequenz:

| | | | | | | | | | | | | | | | |
|---|---|---|---|---|---|---|---|---|---|---|---|---|---|---|---|
| | | | | 5 | | | | | 10 | | | | | 15 | |
| Met | Cys | Asp | Leu | Pro | Gln | Asn | His | Gly | Leu | Leu | Ser | Arg | Asn | Thr | |
| ATG | TGT | GAT | CTG | CCT | CAG | AAC | CAT | GGC | CTA | CTT | AGC | AGG | AAC | ACC | 45 |
| | | | | 20 | | | | | 25 | | | | | 30 | |
| Leu | Val | Leu | Leu | His | Gln | Met | Arg | Arg | Ile | Ser | Pro | Phe | Leu | Cys | |
| TTG | GTG | CTT | CTG | CAC | CAA | ATG | AGG | AGA | ATC | TCC | CCT | TTC | TTG | TGT | 90 |
| | | | | 35 | | | | | 40 | | | | | 45 | |
| Leu | Lys | Asp | Arg | Arg | Asp | Phe | Arg | Phe | Pro | Gln | Glu | Met | Val | Lys | |
| CTC | AAG | GAC | AGA | AGA | GAC | TTC | AGG | TTC | CCC | CAG | GAG | ATG | GTA | AAA | 135 |
| | | | | 50 | | | | | 55 | | | | | 60 | |
| Gly | Ser | Gln | Leu | Gln | Lys | Ala | His | Val | Met | Ser | Val | Leu | His | Glu | |
| GGG | AGC | CAG | TTG | CAG | AAG | GCC | CAT | GTC | ATG | TCT | GTC | CTC | CAT | GAG | 180 |
| | | | | 65 | | | | | | | | | | | |
| Met | Leu | Gln | Gln | Ile | Ser | | | | | | | | | | |
| ATG | CTG | CAG | CAG | ATC | TCA | TGA | TTT | CTG | CTC | TGA | CAA | CCT | CCC | AGG | 225 |
| CAC | AAG | GGC | TGT | ATT | TCT | TCT | CTT | TCA | GAT | AGA | GAG | TGA | TTC | TTT | 270 |
| GGA | AGT | ATT | TCC | TCA | CAG | CCA | GAA | TGG | AGT | CCT | CCT | TCA | TCA | GGG | 315 |
| GAG | TCT | CTG | TCA | CCC | CCA | CCC | CCT | GTA | TCA | CAC | AGG | CTT | CCA | GGT | 360 |
| CAT | TCA | GCT | GCT | GGT | AGA | GTT | CAG | TGT | AGA | ATT | TGT | CTA | GGA | GGG | 405 |
| TCT | CAT | CCC | AAG | CAG | CAG | ATG | AGT | CCT | TTG | TGC | TGA | AGA | GAT | TGA | 450 |
| AGA | TCT | TTT | TCT | TTG | TCA | ACA | AAC | TTG | CAA | GAA | AGT | TTA | AGA | AGT | 505 |
| AAG | GAA | TGA | | | | | | | | | | | | | 514 |

Zur Replikation bzw. zur Expression können die neuen Plasmide der vorliegenden Erfindung in einen bakteriellen Wirt eingebracht werden. Hierbei haben sich Prokaryoten wie E. coli K12, Stamm 294 (ATCC Nr. 31.446), E. coli X1776 (ATCC Nr. 31.537), E. coli W3II0 ($F^-$, Lambda$^-$, Prototroph, ATCC Nr. 27.325), E. coli HBI0I (($F^-$, hsdS20($r^-$, $m^-$), recA13, ara-14, proA2, lacY1, galK2, rpsL20(Smr), xyl-5, mtl-I, supE44,

lambda$^-$), Bazillen wie Bacillus subtilis, und andere Enterobacteriaceae, wie Salmonella typhimurium oder Serratia marcenses und verschiedene Pseudomonaden als geeignet erwiesen.

Zur Expression der neuen Hybridinterferone wurde beispielsweise das Plasmid pRH72, pRH78f und pRH78r jeweils in E. coli transformiert und kultiviert.

Die für die Durchführung der vorliegenden Anmeldung erforderlichen Basisplasmide sind in Sachen der EP-A-0.115.613 bzw. der EP-A-0.170.204 bei der Deutschen Sammlung für Mikroorganismen hinterlegt worden, z.B.
pER103 unter der DMS-Nr. 2773 am 20. Dezember 1983,
E76E9 unter der DMS-Nr. 3003 und
P9A2 unter der DMS-Nr. 3004 jeweils am 4. Juli 1984,
wobei für diese Klone bereits die erforderliche Freigabeerklärung abgegeben wurde. Unter Verwendung dieser Plasmide ist es dem Fachmann auf diesem Gebiet ohne weiteres möglich den Gegenstand der vorliegenden Erfindung an Hand der EP-A-0.115.613 und der EP-A-0.170.204 (siehe auch Nucleic Acids Res. 13, 4739-4749 (1985)) nachzuarbeiten.

Die nachfolgenden Beispiele sollen die Erfindung näher erläutern, ohne diese jedoch einzuschränken:

Vorbemerkung

Alle Enzymreaktionen werden unter den vom Hersteller angegebenen Bedingungen durchgeführt.

Beispiel 1:

Herstellung von parpATER33

10 µg parpER33 werden in 200 µl Reaktionslösung mit je 20 Einheiten BamHI und PstI geschnitten. Anschließend werden die zwei entstehenden Fragmente in einem 1%igen Agarosegel aufgetrennt (1% Agarose in Ix TBE-puffer: 10,8 g/l Tris(hydroxymethyl)aminomethan (Tris), 5,5 g/l Borsäure, 0,93 g/l Äthylendinitrilo-tetraessigsäure-dinatriumsalz (EDTA) und 0,5 mg/l Äthidiumbromid (EtBr), Laufpuffer ist Ix TBE; Elektrophorese bei ca. 5V/cm; Sichtbarmachen der DNA-Fragmente durch Bestrahlung des Agarosegels mit UV-Licht (254 nm)). Das kleinere, das Interferon-alpha2-Arg (IFN-$\alpha$2(Arg)) enthaltende Fragment wird isoliert (Elektrophorese der DNA-Bande auf DE-81 Papier (Whatman), Waschen des Papiers mit 200 mM NaCl, 25 mM Tris pH = 7,5, 1 mM EDTA und Elution der DNA mit 1 M NaCl, 25 mM Tris, pH = 7,5, 1 mM EDTA) und durch Zusatz von 2,5 Vol Äthanol wird die DNA ausgefällt. Nach dem Abzentrifugieren wird die DNA getrocknet und in einem geeigneten Volumen TE-Puffer (10 mM Tris pH = 8,0, 1 mM EDTA) aufgenommen.

10 µg pAT153 werden ebenfalls in 200 µl Reaktionslösung mit je 20 Einheiten BamHI und PstI geschnitten, und die zwei entstehende Fragmente separiert. Von pAT153 isoliert man das größere, den Replikationsursprung enthaltende Fragment.

Jeweils 0,5 µg der gereinigten DNA Fragmente werden in 20 µl Reaktionslösung (66 mM Tris, pH = 7,5, 100 mM NaCl, 10 mM $MgCl_2$, 5 mM Dithiothreit (DTT), 1 mM EDTA, 1 mM Adenosintriphosphat (ATP)) mit 5 Einheiten $T_4$ DNA-Ligase ligiert. Anschließend werden 150 µl kompetente E.coli HB l0l-Bakterien ($F^-$, hsdS20($r^-$, $m^-$), recA13, ara-14, proA2,
lacY1, galK2, rpsL20(Smr), xyl-5, mtl-l, supE44, lambda$^-$) mit 1 µl der Ligasereaktion versetzt, 30 Minuten bei 0°C inkubiert, und durch 2 Minuten 42°C Inkubation mit der DNA transformiert (Kompetente E.coli Bakterien: E.coli wird in LB-Medium (10 g/l Trypton, 5 g/l Yeast Extract, 5 g/l NaCl, pH = 7,5) bis zu $OD_{600nm}$ = 0,3 wachsen gelassen und abzentrifugiert. Die Bakterien werden in 0,5 Vol eiskalter 50 mM $CaCl_2$ Lösung resuspendiert und 30 Minuten inkubiert. Nach erneutem Abzentrifugieren werden die Bakterien in 1/15 Vol des Ausgangsvolumens 50 mM $CaCl_2$ resuspendiert). Die Bakteriensuspension wird auf LB-Agar (LB-Medium plus 15 g/l Agar) mit 50 µg/ml Ampicillin plattiert. Nach 16 Stunden Inkubation bei 37°C werden 12 der entstandenen Kolonien ausgewählt und von diesen im Mikromaßstab die Plasmide isoliert (Birnboim, H.C. and Doly, J., Nucl. Acids Res. 7, 1513 (1979)). Die Richtigkeit der Konstruktion wird durch Restriktionsenzymdoppelverdauung mit PstI-BamHI, PstI-PvuII und EcoRI-BamHI und mit anschließender Gelelektrophorese durch das Auftreten der erwarteten Fragmente bestätigt. Ein Plasmid wird ausgewählt und mit parpATER33 bezeichnet. E.coli transformiert mit parpATER33 zeigt den Phänotyp $Ap^r$ (Ampicillin-Resistenz), $Tc^r$ (TetracyclinResistenz).

Beispiel 2

Herstellung von pRHW14

10 µg parpATER33 werden in 150 µl Reaktionslösung mit HindIII und BamHI doppelt verdaut. Die drei resultierenden DNA Fragmente werden auf einem 1%igen Agarosegel aufgetrennt, und das größte, ca. 3750 Basenpaare (bp) lange Fragment isoliert (Fragment a)). Dieses Fragment trägt den Tryptophan Promotor/Operator (Serratia marcescens), den Replikationsursprung, sowie das $Ap^r$-Gen.
10 µg pRHW12 werden ebenfalls in 150 µl Reaktionslösung mit BamHI und HindIII doppelt verdaut. Die zwei entstehenden Fragmente werden gelelektrophoretisch aufgetrennt, und das kleinere, ca. 800 bp lange Fragment isoliert, welches das IFN-omegal(Gly)-Gen enthält (Fragment b)).
40 ng Fragment a) werden mit ca. 50 ng Fragment b) in 10 µl Reaktionslösung mit 5 Einheiten $T_4$ DNA-Ligase ligiert. 200 µl kompetenter E.coli HBl0l Suspension werden mit der Ligasereaktionslösung gemischt, die Bakterien durch Hitzeschock auf 42°C transformiert und auf LB-Agar plus 50 µg/ml Ampicillin plattiert. Nach 16 Stunden Inkubation bei 37°C werden sechs Kolonien ausgewählt, und aus den Bakterien im Mikromaßstab die Plasmid DNA isoliert. Nach dem Verdauen der DNA mit den Restriktionsendonukleasen EcoRI, HindIII, NcoI bzw. PstI und anschließender gelelektrophoretischer Analyse der Fragmente zeigte sich, daß eines der Plasmide die gewünschte Struktur aufweist. Dieses Plasmid wird mit pRHW14 bezeichnet. E.coli transformiert mit pRHW14 zeigt den Phänotyp $Ap^r$ und $Tc^s$.

Beispiel 3

Nachweis der Plasmid-kodierten Proteine

Der Nachweis Plasmid-kodierter Proteine ist im Maxizell System möglich (Sancar. A., Hack, A.M. and Rupp, W.D., J.Bacteriol. 137, 692-693 (1979)).
Hierzu wird E.coli CSR603 ($F^-$, thr-l, leuB6, proA2, phr-l, recA1, argE3, thi-l, uvrA6, ara-14, lacY1, galK2, xyl-5, mtl-l, gyrA98 (nalA98), rpsL31, tsx-33, $lambda^-$, supE44), transformiert mit den Plasmiden pRHW12 und pRHW14, im Expressionsmedium (10 g/l $(NH_4)_2PO_4$, 3,5 g/l $KH_2PO_4$ pH=7,3, 0,5 g/l NaCl, 21 g/l Caseinhydrolysat (säurehydrolysiert, vitaminfrei), 11 g/l Glucose, 1 mM $MgSO_4$, 0,1 mM $CaCl_2$, 1 mg/l Thiamin-HCl, 20 mg/l L-Cystein, 100 mg/l Ampicillin) bis zu einer Dichte von $OD_{600}$nm=0,6 bei 37°C gezüchtet. 10 ml Kultur werden in einer offenen Petrischale mit einer UV-Germicidlampe (15W) aus 50 cm Entfernung 5 Sekunden lang bestrahlt und eine Stunde weiter inkubiert. Den Kulturen werden 100 µg D-Cycloserin zugesetzt, um noch vermehrungsfähige Bakterien abzutöten. Nach 16 Stunden Inkubation bei 37°C werden die Bakterien abzentrifugiert, zweimal mit je 5 ml Hershey-Salzlösung gewaschen (5,4 g/l NaCl, 3,0 g/l KCl, 1,1 g/l $NH_4Cl$, 15 mg/l $CaCl_2x2H_2O$, 0,2 g/l $MgCl_2x6H_2O$, 0,2 mg/l $FeCl_3x6H_2O$, 87 mg/l $KH_2PO_4$, 12,1 g/l Tris pH=7,4), in 5 ml Hershey-Medium (pro 100 ml Hershey-Salze: 2,0 ml 20% Glucose, 0,5 ml 2% Threonin, 1,0 ml 1 % Leucin, 1,0 ml 2% Prolin, 2% Arginin, 0,1 ml 0,1% Thiamin-HCl) plus 20 µg/ml Indolacrylsäure (IAA) inkubiert. Durch Zusatz von 5 µCi/ml $^{35}$S-Methionin und weiterer Inkubation bei 37°C für eine Stunde werden neusynthetisierte Proteine radioaktiv markiert. Die Bakterien werden abzentrifugiert und in 200 µl Na-Dodecylsulfat(SDS)-Probenpuffer (6,6 mM Na-Phosphat pH=6,8, 2 mM EDTA, 2% SDS, 3% Glyzerin, 0,02% Bromphenolblau, 0,66% 2-Mercaptoäthanol) 5 Minuten bei 100°C lysiert. Die Proben werden anschließend in einem 15%igem Polyacrylamidgel aufgetrennt (Trenngel: 15% Acrylamid, 0,4% Bisacrylamid, 375 mM Tris pH=8,8, 2 mM EDTA, 0,1% SDS; Sammelgel: 6% Acrylamid, 0,16% Bisacrylamid, 375 mM Tris pH=6,8, 2 mM EDTA, 0,1% SDS; Elektrodenpuffer: 3,0 g/l Tris, 14,24 g/l Glyzin, 0,335 g/l EDTA, 0,5 g/l SDS; Elektrophoresedauer: 16 Stunden bei konstant 20 mA). Das Gel wird eine Stunde in 20% Methanol, 7,5% Essigsäure fixiert, 30 Minuten in 5% Methanol, 1% Glyzerin inkubiert und in einem Geltrockner getrocknet. Das Gel wird unter Verwendung einer Verstärkerfolie (Kodak) bei -80°C auf Kodak X-Omat S Röntgenfilm exponiert.
Das Ampicillin-Resistenzgenprodukt (β-Lactamase) wird in beiden Fällen gleich stark markiert. IFN-omegal ist jedoch im Falle des pRHW14 mehr als doppelt so stark markiert als bei pRHW12 (siehe Figur 3).

Beispiel 4

Extraktion von IFN-omegal(Gly) aus Bakterien

E.coli HBl0l transformiert mit pRHW12 bzw. pRHW14 wird in Expressionsmedium plus 20 µg/ml IAA bis zu einer $OD_{600}$nm=20 angezüchtet. Die Bakterien werden durch Zusatz von $H_2SO_4$ bis pH=2 und 60-minütige Inkubation bei 20°C abgetötet. Die Bakterien werden abzentrifugiert, und die Biomasse bei -20°C bis zur Aufarbeitung eingefroren. Die Bakterien werden in 10 Vol 1% Essigsäure resuspendiert. Der pH-Wert der Lösung wird durch Zusatz von 2 N NaOH auf 10 eingestellt, und die Suspension bei 0°C zwei Stunden gerührt. Danach wird mit 2 N HCl wieder ein pH-Wert von 7,5 eingestellt und die Zelltrümmer abzentrifugiert (J2-21 Zentrifuge (Beckman), JA10 Rotor, 4°C, 10000 rpm, 30 Minuten). Die Interferon-Aktivität im Überstand (Rohextrakt) wird mittels des Cytopathischen Effekt (CPE) Reduktionstests auf humanen A549 Zellen (menschliche Lungenkarzinom Zellinie), mit Encephalomyocarditis (EMC) Virus infiziert, unter Verwendung von IFN-α2(Arg) als Standard gemessen. Dabei liefert die Biomasse von E.coli transformiert mit pRHW12 100.000 Einheiten/Gramm (Mittelwert aus drei unabhängigen Züchtungen), während der Klon pRHW14 200.000 Einheiten/Gramm Biomasse (15 Züchtungen) liefert.

Beispiel 5

Konstruktion des Hybridinterferon Expressionsklons pRH72

10 µg parpATER33 bzw. pRHW14 werden in je 130 µl Lösung mit BglII und SphI doppelt verdaut. Die entstandenen Fragmente werden auf einem 0,8% Agarosegel aufgetrennt, die DNA's eluiert und durch Präzipitation gereinigt. Die Fragmente werden in 20 µl TE gelöst. Das Expressionsplasmid für IFN-α2/omegal(BglII) wird durch Ligasereaktion von 1 µl großem Fragment aus parpATER33 mit 5 µl kleinem Fragment von pRHW14 in insgesamt 20 µl unter Verwendung von 5 Einheiten $T_4$ DNA-Ligase hergestellt. Nach der Transformation von E.coli HBl0l werden die Plasmide einiger der resultierenden, Ampicillin resistenten Klone im Mikromaßstab isoliert, und die Richtigkeit der Konstruktion durch doppelte Restriktionsenzymverdauung mit BglII/SphI überprüft. Eines der Plasmide wird ausgewählt und mit pRH72 bezeichnet. E.coli transformiert mit diesem Plasmid besitzt den Phänotyp $Ap^r$,$Tc^s$.

Beispiel 6

Konstruktion der Plasmide pRH78r und pRH78f

1 µl des großen Fragments von pRHW14 wird mit 5 µl des BglII(2)-SphI-Fragments aus parpATER33, das den C-Terminus des IFN-α2(Arg) kodiert, in 20 µl Reaktionslösung mit 5 Einheiten $T_4$ DNA-Ligase ligiert. Mit dem entstehenden Plasmid wird E.coli HBl0l transformiert und wie in Beispiel 5 beschrieben, ein Plasmid der gewünschten Konstruktion ausgesucht. Dieses Zwischenplasmid wird mit pRH77 bezeichnet. Um das Gen für das Hybridinterferon-omegal/α2(BglII) zu komplettieren, muß das beim Schnitt von parpATER33 mit BglII entfernte Fragment in pRH77 eingebracht werden. Hierzu werden 10 µg pRH77 in 50 µl Lösung mit BglII geschnitten, das Volumen der Lösung mit 2x CIP-Puffer verdoppelt (CIP: Kalbsdarm-Phosphatase, 2x CIP-Puffer: 100 mM Tris pH=9,0, 2 mM $MgCl_2$, 0,2 mM $ZnCl_2$), und das 5'-terminale Phosphat durch Zusatz von 1 Einheit CIP (Boehringer Mannheim) entfernt (60 Minuten 37°C). Die linearisierte Form von pRH77 wird durch Agarosegelelektrophorese und Elution der DNA mit anschließender Präzipitation gereinigt. Die DNA wird in 20 µl TE-Puffer aufgenommen und 1 µl davon mit 5 µl des 263 bp Fragments (BglII(1)-BglII(2)-Fragment), das beim Verdauen des parpATER33 mit BglII/SphI gewonnen wird, in insgesamt 10 µl Reaktionslösung mit 5 Einheiten $T_4$ DNA-Ligase ligiert. E.coli HBl0l wird transformiert, und die DNA von sechs entstandenen Kolonien analysiert. Da die Insertion des 263 bp Fragments auf Grund der identischen Enden in zwei Orientierungen erfolgen kann, werden die Plasmide mit den Restriktionsendonukleasen AluI und HaeIII auf die Richtigkeit der Konstruktion hin untersucht. Das Plasmid, in dem das BglII-Fragment in der für die Expression richtigen Lage insertiert wurde, wird mit pRHW78r, jenes mit der für Expression falschen Orientierung mit pRH78f bezeichnet. E.coli transformiert mit pRH78r bzw. pRH78f zeigt den Phänotyp $Ap^r$, $Tc^r$.

Beispiel 7

Lysattest zur Feststellung von Interferon-(antiviraler) Aktivität

E.coli transformiert mit den diversen Plasmiden wird in 35 ml Expressionsmedium plus 20 µg/µl IAA bei 37°C bis zu einer $OD_{600nm}$ = 0,6 angezüchtet. Die Bakterien werden abzentrifugiert. Das Bakterienpellet wird in 3,5 ml 50 mM Tris pH=7,6/30 mM $MgCl_2$-Lösung aufgenommen und unter Eiskühlung mit einem Ultraschall Desintegrator (MSE, Soniprep 150) mit maximaler Leistung beschallt. Die Suspension wird 10 Minuten zentrifugiert (J2-21 Zentrifuge, 10.000 rpm, 4°C, JA20-Rotor), und der Überstand steril filtriert. Die antivirale Aktivität der Lösung wird mittels des CPE-Reduktionstests (A549-Zellen, EMC-Virus) bestimmt.
E.coli transformiert mit pRH72 (IFN-α2/omegal(BglII)) ergibt keine antivirale Aktivität ebenso wie E.coli

transformiert mit pRH78f, das die ersten 64 Aminosäuren von reifen IFN-omegal, gefolgt von einem Serin kodiert.

Demgegenüber zeigt das IFN-omegal/α2(BglII), wobei der Klon pRH78r ca. 30x10$^6$ Einheiten Interferon (verglichen mit IFN-α2(Arg) als Standard) pro Liter Kultur und pro 1 $OD_{600}$nm Bakteriendichte produziert, eine etwa viermal höhere spezifische antivirale Aktivität auf A549 Zellen als IFN-α2(Arg).

In der folgenden Tabelle sind die Aminosäureänderungen des Hybridinterferons IFN-omegal/α2(BglII) gegenüber IFN-α2(Arg) aufgelistet:

| Position | IFN-α2(Arg) | IFN-omegal/α2 | Übergang |
|---|---|---|---|
| 7 | Thr | Asn | p >> p |
| 9 | Ser | Gly | p >> p |
| 11 | Gly | Leu | p >> l |
| 14 | Arg | Asn | b >> p |
| 17 | Met | Val | (l) >> l |
| 20 | Ala | His | l >> b |
| 27 | Leu | Pro | l >> l |
| 29 | Ser | Leu | p >> l |
| 38 | Gly | Arg | p >> b |
| 43 | Glu | Met | a >>(l) |
| 44 | Phe | Val | ar >> l |
| 45 | 0 | Lys | 0 >> b |
| 47 | Asn | Ser | p >> p |
| 49 | Phe | Leu | ar >> l |
| 53 | Glu | His | a >> b |
| 54 | Thr | Val | p >> l |
| 55 | Ile | Met | l >>(l) |
| 56 | Pro | Ser | l >> p |
| 62 | Ile | Leu | l >> l |

Legende:

a: sauer, ar: aromatisch, b: basisch, l: apolar, p: polar

0: keine Aminosäure an dieser Position

Unterschiede bei den Ladungen (Position):

1) Verlust von 1 positiven Ladung: 14
2) Gewinn von 4 positiven Ladungen: 20, 38, 45, 53
3) Verlust von 2 negativen Ladungen: 43, 53

Das Hybrid hat 5 positive Ladungen mehr als IFN-α2(Arg).

Beispiel 9:

Reinigung des IFN-omegal/α2(BglII)

145 g säurebehandelte und bei -20°C gefrorene E.coli des Klones HB l0l/pRH78r werden in 1450 ml 1% Essigsäure bis zur völligen Verteilung des Materials unter Eiskühlung gerührt (etwa 30 Minuten) und sodann 2 x 1 Minute lang mit dem Ultra-Turrax T 45/6 (Janke und Kunkel) bei 10.000 UpM homogenisiert. Anschließend wird Polymin P (Serva, Kat.Nr. 33141) bis zu einer Konzentration von 0,25% zugegeben und der pH mit 5 N NaOH auf 10,0 eingestellt. Nach 2 Stunden Rühren unter Eiskühlung wird der pH mit 5 N HCl auf 7,5 eingestellt und eine Klärung des Rohextraktes durch Zentrifugation vorgenommen (Christ Cryofuge 6-6 S, 3000 UpM, 1 Stunde, etwa 4°C).

Zur Fällung des Interferons wird der Rohextrakt mit 430 g/Liter Ammoniumsulfat versetzt und zur vollständigen Fällung 16 Stunden bei 4-8°C stehen gelassen. Der Niederschlag wird dann durch Zentrifugation gewonnen (Beckman Zentrifuge J 2-21, Rotor JA10, 10.000 UpM, 60 Minuten, 4-8°C). Das Ammonsulfat-Pellet wird in 145 ml 0,01 M NaCl aufgenommen und die Suspension mit 5 N NaOH auf pH 7,5 eingestellt. Nach 3 Stunden Rühren (Eiskühlung) wird die Lösung klarzentrifugiert (Beckman J2-21, Rotor JA10, 10 000 UpM, 4°C, 60 Minuten) und mit Hilfe einer Nephross-Allegro Dialysierpatrone (Fa. Organon) gegen 0,01 M NaCl solange dialysiert, bis die Interferonlösung eine Osmolarität von 370 mOsmol/l aufwies. Tandem-Chromatographie: Zur chromatographischen Reinigung wird eine Säule aus 75 g DE-52 Zellulose (Whatman) mit 0,025 M Tris/HCl + 0,2 M NaCl, pH 7,5 äquilibriert und vor eine Affinitätssäule (60 ml) vorgeschaltet, die den monoklonalen Antikörper EBI-l (siehe EP-A-0.119.476), gekuppelt an Sepharose 4B (mit Hilfe von BrCN-aktivierter Sepharose 4B, Pharmacia, hergestellt) enthält. Die Affinitätssäule enthält 480 mg des monoklonalen Antikörpers EBI-l, sie wird ebenfalls mit Tris/HCl + NaCl pH 7,5 wie oben beschrieben äquilibriert. Die Interferonlösung wird durch die beiden Säulen gepumpt und solange mit 0,025 M Tris/HCL + 0,2 M NaCl nachgewaschen, bis im Eluat nur mehr eine Extinktion $OD_{280}$nm von unter 0,1 gemessen wird. Danach wird die Vorsäule (DE-52 Zellulose) abgekuppelt, und die EBI-l Säule solange weiter gewaschen, bis das Eluat proteinfrei ist ($OD_{280}$nm im Eluat unter 0,01). Das adsorbierte Interferon wird nunmehr mit Hilfe von 0,1 M Zitronensäure in 25%igem Äthylenglykol eluiert. Der Proteinpeak wird gesammelt.

Das saure Eluat der Antikörpersäule wird mit 2 N Ammoniak auf pH 4,5 eingestellt, und der entstandene Niederschlag abzentrifugiert.

Die letzte Reinigungstufe ist eine Ionenaustausch-Chromatographie an dem Träger MONO-S (Pharmacia). Eine Säule von 1 ml Bettvolumen (HR 5/5) wird an eine FPLC-Apparatur angeschlossen (Pharmacia) und in 0,1 M Na-Citrat pH 4,2 äquilibriert. Die IFN-Lösung wird aufgetragen, und das adsorbierte Interferon mit Hilfe eines pH-Gradienten eluiert (Puffer A: 0,1 M Na-Citrat pH 4,2 und Puffer B: 0,1 M Na-Phosphat pH 8,0). Das Interferon-omegal wird bei einem pH von 7,0 eluiert und der IFN-Peak gesammelt (siehe Fig.6).

Übersicht über die Reinigung von IFN-omegal/α2(BglII)

Ausgangsmaterial: 145 g E.coli HB l0l/pRH78r

| | Vol. (ml) | IFN[x)] (Einh.) | Protein[xx)] (mg) | Einh. /mg | Ausb. (%) |
|---|---|---|---|---|---|
| Rohextrakt | 1470 | $16,9x10^9$ | 3000 | $5,6x10^6$ | 100 |
| nach Fällung Ammonsulfat u. Dialyse | 246 | $14,0x10^9$ | 1970 | $7,1x10^6$ | 83 |
| Eluat der Antikörpersäule | 11,1 | $10,3x10^9$ | 12,9 | $800x10^6$ | 61 |
| Überstand pH 4,5 | 11,9 | $7,4x10^9$ | 9,3 | $800x10^6$ | 44 |
| Pool nach MONO-S | 6,9 | $4,6x10^9$ | 7,0 | $660x10^6$ | 27 |

x) Die Bestimmung des Interferon-Gehalts erfolgt durch Messung

der antiviralen Aktivität (CPE-Reduktionstest) mit Hilfe von A549-Zellen und Encephalomyocarditis-Virus (EMC-Virus). Als Standard dient IFN-α2(Arg).

xx) Die Proteinbestimmung erfolgt mit Hilfe des Bio-Rad Protein Assays. Der Standard war Serumalbumin.

Beurteilung der Reinigung

Das gereinigte Hybridinterferon-omegal/α2(BglI) ist homogen (siehe hierzu Fig. 7 - Gelpermeations-HPLC). Die erreichte spezifische Aktivität von etwa $800 \times 10^6$ Einheiten/mg Protein ist höher als die von IFN-α2(Arg) (ca. $300 \times 10^6$ Einheiten/mg Protein).

Beispiel 10

Herstellung eines monoklonalen Antikörpers gegen HuIFN-omegal

a) Immunisierung:

Zwei weibliche, etwa acht Wochen alte Balb/c-Mäuse werden mit hochgereinigtem IFN-omegal/α2(BglII) (Reinheit >95%, gelöst in O.1 M Natriumphosphat/Natriumcitrat pH 7) wie folgt immunisiert:

1. Immunisierung: 100 µg IFN-omegal/α2(BglII) in einer Emulsion mit komplettem Freund's Adjuvans, intraperitoneal injiziert
2. Immunisierung: 100 µg IFN-omegal/α2(BglII) in einer Emulsion mit inkomplettem Freund's Adjuvans, ein Monat nach der ersten Immunisierung intraperitoneal injiziert

Zehn Tage nach der zweiten Immunisierung werden Blutproben gewonnen. Die Fähigkeit der Seren, die antivirale Aktivität von HuIFN-omegal, HuIFN-α2(Arg) und IFN-omegal/α2(BglII) zu neutralisieren, wird wie folgt getestet:
100 µl einer Verdünnung der Serumprobe in Zellkulturmedium werden mit 100 µl einer IFN-Losung (jeweils 100 antivirale Einheiten/ml) in Zellkulturmedium gemischt und 90 Minuten bei 37 °C inkubiert. Anschließend wird die antivirale Aktivität der Proben im biologischen Test (A549 Lungenkarzinomzellen, Encephalomyocarditis Virus) bestimmt:

| Serumverdünnung | Maus 1 | | Maus 2 | | | |
|---|---|---|---|---|---|---|
| | IFN-Präparat | | | | | |
| omegal/ | omegal | α2(Arg) | omegal/α2 | omegal | α2(Arg) | α2 |
| 1: 100 | + | + | + | – | – | + |
| 1: 1 000 | – | – | + | – | – | +/– |
| 1:10 000 | – | – | – | – | – | – |

Symbole: + vollständige Neutralisierung, +/- partielle Neutralisierung, - keine neutralisierende Wirkung

Vier Wochen nach der zweiten Immunisierung erhält Maus 1 eine intravenöse Injektion von weiteren 100 µg IFN-omegal/α2(BglII).Drei Tage später wird die Milz entnommen und zur Herstellung von Hybridomen eingesetzt.

Drei Wochen nach der zweiten Immunisierung wird Maus 2 neuerlich immunisiert; 100 µg IFN-omegal/α2-(BglII) werden intraperitoneal, 100 µg IFN-omegal/α2(BglII) subkutan appliziert. Zwei Wochen später wird eine weitere Serumprobe gewonnenen, die im oben beschriebenen Test bei einer Verdünnung von 1:100 eine partiell neutralisierende Wirkung für HuIFN-omegal zeigt. Vier Wochen nach der dritten Immunisierung wird eine intravenöse Injektion von 100 µg IFN-omegal/α2(BglII) gegeben, vier Tage später die Milz entnommen und zur Herstellung von Hybridomen eingesetzt.

b) Herstellung und Screening von Hybridomen

Die Herstellung von Hybridomen erfolgt nach der ursprünglich von Köhler und Milstein (Nature 256, 495 (1975)) entwickelten Methode unter Verwendung der nicht-sezernierenden Zellinie P3X63Ag8.653 (Kearney et al., J.Immunol. 123, 1548 (1979)). Zur Fusion wird 50% Polyäthylenglykol 4000 mit 5% Dimethylsulfoxid eingesetzt. Die Selektion der Hybridzellen in HAT-Medium erfolgt ebenfalls mit Hilfe bekannter Verfahren (siehe Monoclonal Antibodies: Production and Maintenance, Lovborg, U. William Heinemann Medical Books, London 1982; Monoclonal Antibodies, Hybridomas: A New Dimension in Biological Analyses, Kennet, R.H., McKearn, T.J., and Bechtol, K.B., Herausgeber: Plenum Press, New York and London 1980). Aus beiden Fusionen werden insgesamt 730 Hybridomkulturen erhalten. Das Screening wird wie folgt durchgeführt: Kulturüberstände von mindestens 10-20% konfluenten Hybridomkulturen werden mit gleichen Volumina einer Lösung von HuIFN-omegal (20 antivirale Einheiten/ml) gemischt, 90 Minuten bei 37°C inkubiert und anschließend auf ihre antivirale Aktivität getestet. Alle Kulturen werden in Abständen von jeweils einer Woche mindestens zweifach getestet. Nur eine der 730 Hybridomkulturen zeigt in allen Tests konsistent eine Reduktion der antiviralen Aktivität. Diese Kultur, im folgenden OMG-2 genannt, wird durch limitierende Verdünnung subkloniert, die Subklone erneut im beschriebenen Testverfahren auf Aktivität getestet. Mehrere positive Subklone werden in mit Pristan vorbehandelte Mäuse inokuliert. Einer der Subklone führte in allen Mäusen zur Bildung von Antikörper-enthaltender Ascites-Flüssigkeit. Durch Fällung mit 55% gesättigter Ammonsulfatlösung wird der enthaltene Antikörper partiell gereinigt. Das resultierende Präparat enthält Antikörper in etwa 75%iger Reinheit (bestimmt durch Gelpermeations-Hochdruckflüssigkeitschromatographie). Etwa 10 mg Antikörper kann aus einem Milliliter Ascites-Flüssigkeit gewonnen werden. Eine weitere Reinigung bis zu einer Reinheit von über 95% wird durch Anionenaustausch-Chromatographie erzielt.

c) Charakterisierung des Antikörpers OMG-2

In der Natriumdodecylsulfat-Polyacrylamidgelelektrophorese unter nicht-reduzierenden Bedingungen zeigt der Antikörper OMG-2 ein Molekulargewicht von etwa 150 000. In der Gelpermeations-Hochdruckflüssigkeitschromatographie zeigt der Antikörper ein mit einem IgG-Markerprotein identisches Retentionsverhalten. Der Antikörper ist somit wahrscheinlich vom IgG-Typ.
Im Neutralisationstest (siehe Beispiel 10b) werden mit einem durch Ammonsulfat-Fällung partiell (siehe Beispiel 10b) gereinigtem Antikörper folgende Ergebnisse erhalten:

| Antikörper-Konzentration µg/ml | IFN-Präparation | | |
|---|---|---|---|
| | omega1 | alfa2 | omega1/α2 |
| 5 000 | +/- | +/- | |
| 1 000 | - | - | + |
| 100 | - | - | + |
| 10 | - | - | +/- |
| 1 | | | - |

Der Antikörper zeigt somit starke neutralisierende Wirkung gegen das zur Immunisierung der Mäuse eingesetzte Hybrid-IFN-omega1/α2(BglII), aber nur eine etwa 500-fach schwächere Wirkung gegen HuIFN-α2(Arg) und HuIFN-omega1. Trotz der offensichtlich relativ geringen Affinität zu HuIFN-omega1 kann der Antikörper OMG-2 erfolgreich für die Immunaffinitätschromatographie dieses Interferons eingesetzt werden (siehe Beispiel 11).

Beispiel 11

Reinigung von IFN-omega1

a) Extraktion und CPG-Chromatographie:

794 g säuregefällte und bei -20°C tiefgefrorene E.coli des Klones pRHW14 werden in 7700 ml 1%iger Essigsäure unter Eiskühlung bis zur völligen Verteilung des Materials gerührt (etwa 30 Minuten) und mit Hilfe von 2N NaOH auf pH 10 eingestellt. Nach 2-stündigem Rühren unter Eiskühlung wird die Suspension auf pH 7,5 eingestellt (2N HCl) und klarzentrifugiert (1 Stunde bei 10.000 UpM, 4°C, JA 10-Rotor der Beckman Zentrifuge J2-21). Der klare Überstand wird mit 50 ml/Stunde über eine 500 ml-Säule aus CPG gepumpt (controlled pore glass, CPG 10-350, 120-200 mesh, Electro-Nucleonics Inc, USA) und anschließend die Säule gründlich mit 0.025 M Tris/HCl + 1 M NaCl (pH 7,5) nachgewaschen. Das an der Säule adsorbierte Interferon wird sodann mit 0,025 M Tris/HCl + 1 M KSCN in 50%igem Äthylenglykol (pH 7,5) eluiert (50 ml/Stunde). Der IFN-Pool wird sodann gegen 0,025 M Tris/HCl + 0,1 M NaCl dialysiert, wobei durch Zugabe von 10% Polyäthylenglykol 40.000 zu der Außenlösung gleichzeitig eine Konzentrierung des IFN-Pools erreicht wird. Das dialysierte Konzentrat wird durch Zentrifugation geklärt (1 Stunde, 4°C, 15.000 UpM, JA-20 Rotor der Beckman Zentrifuge J2-20).

b) Affinitätschromatographie an OMG 2-Sepharose

Gereinigter monoklonaler Antikörper OMG-2 wird mit Hilfe von BrCN-aktivierter Sepharose 4B nach der Vorschrift des Herstellers (Pharmacia) an den Träger Sepharose 4B gekuppelt. Hierbei werden 16 mg des

monoklonalen Antikörpers pro Gramm aktivierte Sepharose 4B eingesetzt. Für die beschriebene Trennung wird eine Affinitätssäule von 8 ml Volumen verwendet.

Das nach Beispiel 11a erhaltene Konzentrat der CPG-Säule wird mit 4 ml/Minute über die Affinitätssäule gepumpt und die Säule anschließend mit 0,025 M Tris/HCl + 0,1 M NaCl pH 7,5 solange gewaschen, bis das Eluat proteinfrei war ($OD_{280}$nm des Eluates identisch mit der des Waschpuffers). Das gebundene Interferon wird sodann mit 2 ml/Minute mit Hilfe von 0,1 M Zitronensäure in 25% Äthylenglykol eluiert und der Proteinpeak ($OD_{280}$nm) gesammelt.

c) Ionenaustausch-Chromatographie an dem Träger MONO-S

Eingesetzt wird eine 1 ml-Säule (HR 5/5) des Trägermaterials MONO-S (beide Pharmacia), die an eine FPLC-Apparatur (Pharmacia) angeschlossen war. Die Ionenaustauschersäule wird mit 0,1 M K-Phosphat pH 6,0 in 25% Propylenglykol äquilibriert und das nach Beispiel 11b erhaltene Eluat der Affinitätssäule mit 0,5 ml/Minuten durchgepumpt. Das adsorbierte Interferon wird sodann mit Hilfe eines NaClGradienten eluiert (Puffer B: 0,1 M K-Phosphat + 1 M NaCl pH 6,0 in 25% Propylenglykol). Die Fraktionen (je 1 ml) werden auf Interferon-Aktivität getestet. Der erste Peak ($OD_{280}$nm) bei 46% Puffer B enthält das Interferon-omega (siehe Fig. 8).

d) Reverse-Phase-HPLC

Als stationäre Phase dient eine Bakerbond WP-RP 18 Säule, 4 x 250 mm, mit einem Partikeldurchmesser von 5 µm und einem Porendurchmesser von 300 A.
Als mobile Phase dient ein Gradient von Acetonitril in 0,1 in 0,1% Trifluoressigsäure:
Puffer A: 0,1% Trifluoressigsäure in Wasser,
Puffer B: 0,1% Trifluoressigsäure in Acetonitril,
Gradient von 20-68% B in 28 Minuten,
Fließrate: 1 ml/Minute,
Detection: $OD_{214}$nm.

Es werden 630 µg des gemäß Beispiel 11c erhaltenen Proteins (IFN-Pool nach MONO-S) aufgetragen. Das Eluat wird im Bereich von 48-65% B getestet. Das Interferon-omegal eluiert bei einer Retentionszeit von 24,5 Minuten und 63% Puffer-B (siehe Fig. 9). Die Ausbeute beträgt 5 bis 10 µg, die spezifische Aktivität >$10^8$ Einheiten/mg Protein.

e) Bestimmung der N-terminalen Aminosäuresequenz

Der gemäß Beispiel 11d erhaltene IFN-omegal-Peak (RP-HPLC) wird in einer Speedvac-Zentrifuge getrocknet und in einem Sequenzer des Typs 470 A (Applied Biosystems) analysiert. Folgende Aminosäuresequenz wird erhalten:

```
Xxx-Asp-Leu-Pro-Gln-Asn-Xxx-Gly-Leu-Leu-Ser-
1               5                    10
```

Diese Sequenz bestätigt die aus der cDNA abgeleitete Reihenfolge der Aminosäuren (Cystein an der l.Stelle ist ohne Reduktion und Alkylierung des Proteins nicht nachweisbar und Histidin an der 7. Position konnte nicht eindeutig nachgewiesen werden).

Übersicht über die Reinigung von IFN-omegal:

1. Extraktion und CPG Chromatographie

| | Vol. (ml) | IFN x) (Einh.) | Protein xx) (mg) | Einh. /mg | Ausb. (%) |
|---|---|---|---|---|---|
| Rohextrakt | 7700 | $190 \times 10^6$ | 19.100 | 9.950 | 100 |
| Pool nach CPG | 425 | $53 \times 10^6$ | 4.600 | 11.500 | 28 |
| nach Dialyse | 410 | $45 \times 10^6$ | 1.650 | 27.300 | 24 |

x) Die Bestimmung des Interferongehaltes erfolgt durch die Messung der antiviralen Aktivität (CPE-Reduktions-Test) mit Hilfe von A 549-Zellen und Encephalo-myocarditis-Virus (EMC-Virus). Standard: rIFN-α2(Arg). Es handelt sich hierbei um den Mittelwert dreier unabhängiger Tests.

xx) Die Proteinbestimmung erfolgt mit Hilfe des Bio-Rad Protein Assays. Der Standard ist Serumalbumin.

2. Affinitätschromatographie an OMG 2-Sepharose

| | Vol. (ml) | IFN x) (Einh.) | Protein xx) (mg) | Einh. /mg | Ausb. (%) |
|---|---|---|---|---|---|
| aufgetragen | 350 | $40,6 \times 10^6$ | 1.520 | 26.700 | 100 |
| nicht adsorbiert | 440 | $4,5 \times 10^6$ | ----- | ------ | 11 |
| Eluat IFN-omega | 7 | $25,6 \times 10^6$ | 2,84 | $9,0 \times 10^6$ | 63 |

3. Ionenaustausch Chromatographie an MONO-S

| | Vol. (ml) | IFN x) (Einh.) | Protein xx) (mg) | Einh. /mg | Ausb. (%) |
|---|---|---|---|---|---|
| aufgetragen | 7 | $7,14 \times 10^6$ | 2,84 | $2,5 \times 10^6$ | 100 |
| IFN omega-Pool | 2 | $3,04 \times 10^6$ | 0,72 | $4,2 \times 10^6$ | 43 |

**Patentansprüche**

**1.** Monoclonale Antikörper, die die Aktivität von BglII-Hybridinterferonen der allgemeinen Formel

x) Die Bestimmung des Interferongehaltes erfolgt durch die Messung der antiviralen Aktivität (CPE-Reduktions-Test) mit Hilfe von A 549-Zellen und Encephalo-myocarditis-Virus (EMC-Virus). Standard: rIFN-α2(Arg). Es handelt sich hierbei um den Mittelwert dreier unabhängiger Tests.

xx) Die Proteinbestimmung erfolgt mit Hilfe des Bio-Rad Protein Assays. Der Standard ist Serumalbumin.

$$R_1 - \text{Gln Ile Phe} - R_2 \quad ,(I)$$

CAG ATC TTC
BglII

in der
BglII die gemeinsame BglII-Restriktionsstelle der α1-, α2- und omega-Interferone,
$R_1$ die Peptidsequenz eines α1- oder α2-Interferons, die durch die DNA-Sequenz dieser Interferone vor der BglII-Schnittstelle kodiert wird, und $R_2$ die Peptidsequenz eines omega-Interferons, die durch die DNA-Sequenz dieses Interferons nach der BglII-Schnittstelle kodiert wird, oder
$R_1$ die Peptidsequenz eines omega-Interferons, die durch die DNA-Sequenz dieses Interferons vor der BglII-Schnittstelle kodiert wird, und $R_2$ die Peptidsequenz eines α1- oder α2-Interferons, die durch die DNA-Sequenz dieser Interferone nach der BglII-Schnittstelle kodiert wird, bedeuten, deren N-terminale Met- oder N-Formyl-Met-Derivate und, falls die Peptidsequenz des Hybridinterferons eine Glykosylierungsstelle enthält, deren N-glykosylierte Derivate, und die Aktivität von Humaninterferonen des Typs α2 und omegal ganz oder teilweise neutralisieren,

und diese Antikörper produzierende Hybridzellinien.

**2.** Monoclonale Antikörper gemaß Anspruch 1, dadurch gekennzeichnet, daß als BglII-Hybridinterferone Interferone bestehend aus IFN-α2(Arg) und IFN-omegal(Glu) oder IFN-omegal(Gly) verwendet werden, deren N-terminale Met- oder N-Formyl-Met-Derivate und deren N-glykosylierte Derivate.

**3.** Monoclonale Antikörper gemäß Anspruch 2, dadurch gekennzeichnet, daß als Hybridinterferone Interferone der Formel

```
                    5                       10                      15
Cys Asp Leu Pro Gln Thr His Ser Leu Gly Ser Arg Arg Thr Leu

                    20                      25                      30
Met Leu Leu Ala Gln Met Arg Arg Ile Ser Leu Phe Ser Cys Leu

                    35                      40                      45
Lys Asp Arg Arg Asp Phe Gly Phe Pro Gln Glu Glu Phe Gly Asn

                    50                      55                      60
Gln Phe Gln Lys Ala Glu Thr Ile Pro Val Leu His Glu Met Ile

                    65                      70                      75
Gln Gln Ile Phe Ser Leu Phe His Thr Glu Arg Ser Ser Ala Ala

                    80                      85                      90
Trp Asn Met Thr Leu Leu Asp Gln Leu His Thr Gly Leu His Gln

                    95                      100                     105
Gln Leu Gln His Leu Glu Thr Cys Leu Leu Gln Val Val Gly Glu

                    110                     115                     120
Gly Glu Ser Ala -X- Ala Ile Ser Ser Pro Ala Leu Thr Leu Arg

                    125                     130                     135
Arg Tyr Phe Gln Gly Ile Arg Val Tyr Leu Lys Glu Lys Lys Tyr

                    140                     145                     150
Ser Asp Cys Ala Trp Glu Val Val Arg Met Glu Ile Met Lys Ser

                    155                     160                     165
Leu Phe Leu Ser Thr Asn Met Gln Glu Arg Leu Arg Ser Lys Asp

                    170
Arg Asp Leu Gly Ser Ser
```

in der
X für Gly oder Glu steht, verwendet werden, deren N-terminale Met- oder N-Formyl-Met-Derivate und deren N-glykosylierte Derivate.

**4.** Monoclonale Antikörper gemäß Anspruch 2, dadurch gekennzeichnet, daß als Hybridinterferon IFN-omega1/α2(BglII) der Formel

| | | | | | | | | | | | | | | |
|---|---|---|---|---|---|---|---|---|---|---|---|---|---|---|
| | | | | 5 | | | | | 10 | | | | | 15 |
| Cys | Asp | Leu | Pro | Gln | Asn | His | Gly | Leu | Leu | Ser | Arg | Asn | Thr | Leu |
| | | | | 20 | | | | | 25 | | | | | 30 |
| Val | Leu | Leu | His | Gln | Met | Arg | Arg | Ile | Ser | Pro | Phe | Leu | Cys | Leu |
| | | | | 35 | | | | | 40 | | | | | 45 |
| Lys | Asp | Arg | Arg | Asp | Phe | Arg | Phe | Pro | Gln | Glu | Met | Val | Lys | Gly |
| | | | | 50 | | | | | 55 | | | | | 60 |
| Ser | Gln | Leu | Gln | Lys | Ala | His | Val | Met | Ser | Val | Leu | His | Glu | Met |
| | | | | 65 | | | | | 70 | | | | | 75 |
| Leu | Gln | Gln | Ile | Phe | Asn | Leu | Phe | Ser | Thr | Lys | Asp | Ser | Ser | Ala |
| | | | | 80 | | | | | 85 | | | | | 90 |
| Ala | Trp | Asp | Glu | Thr | Leu | Leu | Asp | Lys | Phe | Tyr | Thr | Glu | Leu | Tyr |
| | | | | 95 | | | | | 100 | | | | | 105 |
| Gln | Gln | Leu | Asn | Asp | Leu | Glu | Ala | Cys | Val | Ile | Gln | Gly | Val | Gly |
| | | | | 110 | | | | | 115 | | | | | 120 |
| Val | Thr | Glu | Thr | Pro | Leu | Met | Lys | Glu | Asp | Ser | Ile | Leu | Ala | Val |
| | | | | 125 | | | | | 130 | | | | | 135 |
| Arg | Lys | Tyr | Phe | Gln | Arg | Ile | Thr | Leu | Tyr | Leu | Lys | Glu | Lys | Lys |
| | | | | 140 | | | | | 145 | | | | | 150 |
| Tyr | Ser | Pro | Cys | Ala | Trp | Glu | Val | Val | Arg | Ala | Glu | Ile | Met | Arg |
| | | | | 155 | | | | | 160 | | | | | 165 |
| Ser | Phe | Ser | Leu | Ser | Thr | Asn | Leu | Gln | Glu | Ser | Leu | Arg | Ser | Lys |
| | | | | 170 | | | | | | | | | | |
| Glu | | | | | | | | | | | | | | |

verwendet wird, dessen N-terminales Met- oder N-Formyl-Met-Derivat.

5. Verwendung eines wie in den Ansprüchen 1 bis 4 charakterisierten Hybridinterferons zur Immunisierung von Versuchstieren und zur Herstellung von monoclonalen Antikörpern gemäß den Ansprüchen 1 bis 4.

6. In vitro und in vivo zu kultivierende Hybridzellinien, dadurch gekennzeichnet, daß diese durch Zellfusion von Milzzellen einer mit einem Hybridinterferon gemäß den Ansprüchen 1 bis 4 immunisierten Maus, vorzugsweise einer BALB/c-Maus, mit Myelomzellen, vorzugsweise von Zellen der Linie P3-X-63Ag8-653, erhalten werden und Antikörper gegen diese Hybridinterferone, gegen α- und omegal-Interferon produzieren.

7. Verwendung der monoklonalen Antikörper gemäß Anspruch 1 zur Reinigung von einem Hybridinterferon gemäß den Ansprüchen 1 bis 4, einem IFN-α2 oder einem IFN-omegal.

8. Verfahren zur Herstellung von neuen IgG-Antikörpern der Maus mit Anti-Humaninterferon-α2-, -omegal- und Antihybridinterferonspezifität gemäß Anspruch 1, dadurch gekennzeichnet, daß eine die IgG-Antikörper produzierende Hybridzellinie durch Zellfusion hergestellt und subkloniert wird, wobei zur Zellfusion Myelomzellen und Milzzellen von gegen Hybridinterferonen gemäß den Ansprüchen 1 bis 4 immunisierten Mäusen verwendet werden, und nach Wachstum der Zellen die IgG-Antikörper mit Anti-Interferonspezifität isoliert werden.

**9.** Verfahren zur Herstellung eines Antikörper-Affinitätsträgers, dadurch gekennzeichnet, daß ein monoklonaler Antikörper gemäß Anspruch 1 kovalent an einen aktivierten Trager gebunden ist.

Fig. 1

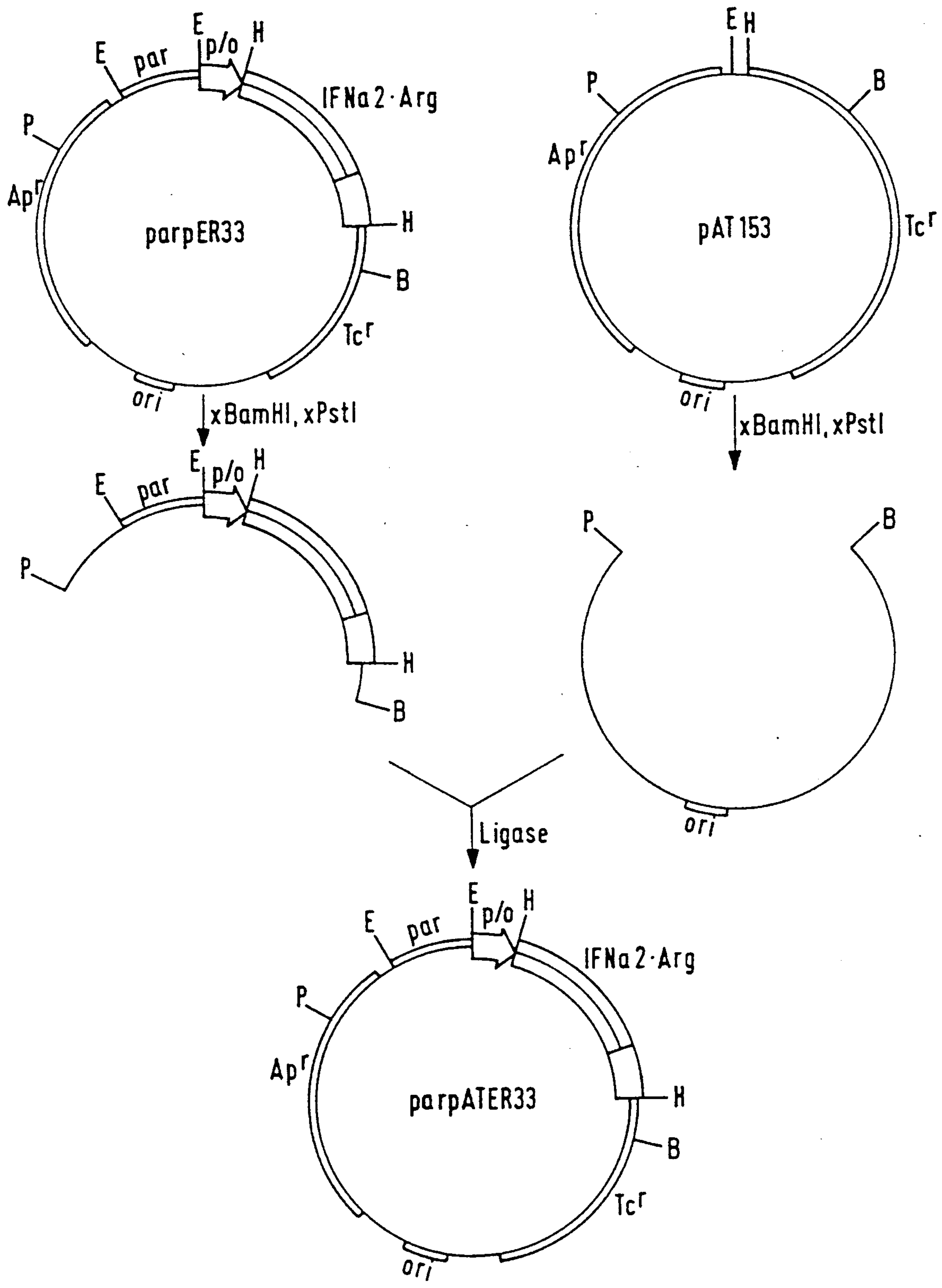
E
par
E
p/o
H
IFNa 2·Arg
P
Ap^r
parpER33
H
B
Tc^r
ori
xBamHI, xPstI
E
H
P
B
Ap^r
pAT 153
Tc^r
ori
xBamHI, xPstI
E
par
E
p/o
H
P
H
B
P
B
ori
Ligase
E
par
E
p/o
H
IFNa 2·Arg
P
Ap^r
parpATER33
H
B
Tc^r
ori

Fig. 2

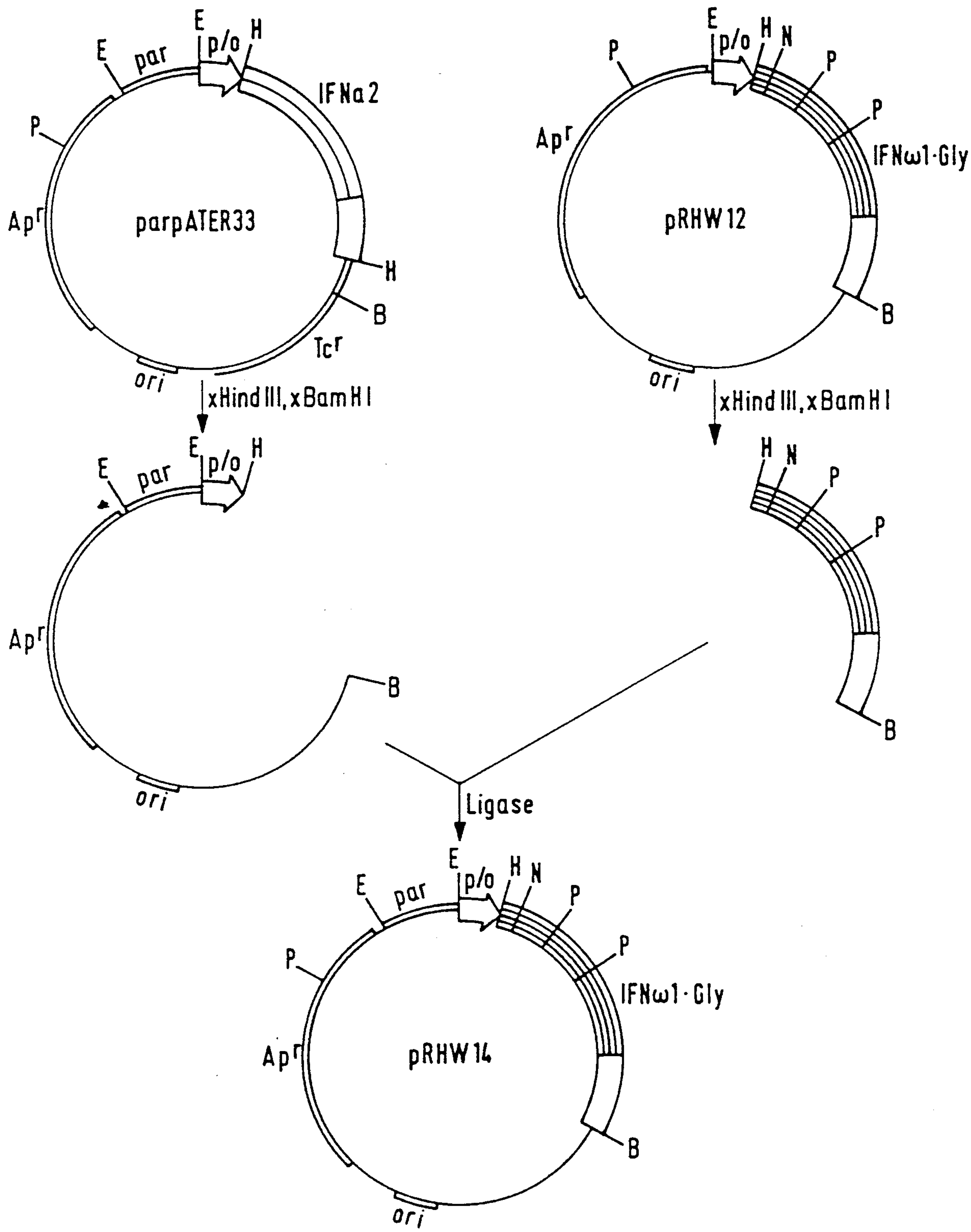
E
par
E
p/o
H
IFNa2
P
Apr
parpATER33
H
B
Tcr
ori
xHindIII, xBamHI
E
par
E
p/o
H
Apr
B
ori
P
E
p/o
H
N
P
P
Apr
IFNω1-Gly
pRHW12
B
ori
xHindIII, xBamHI
H
N
P
P
B
Ligase
E
par
E
p/o
H
N
P
P
P
IFNω1-Gly
Apr
pRHW14
B
ori

Fig. 3

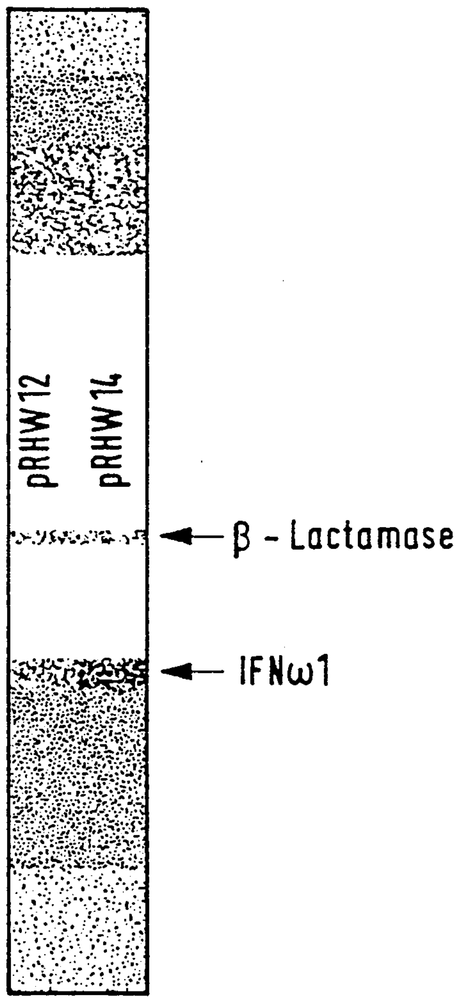

Fig. 4

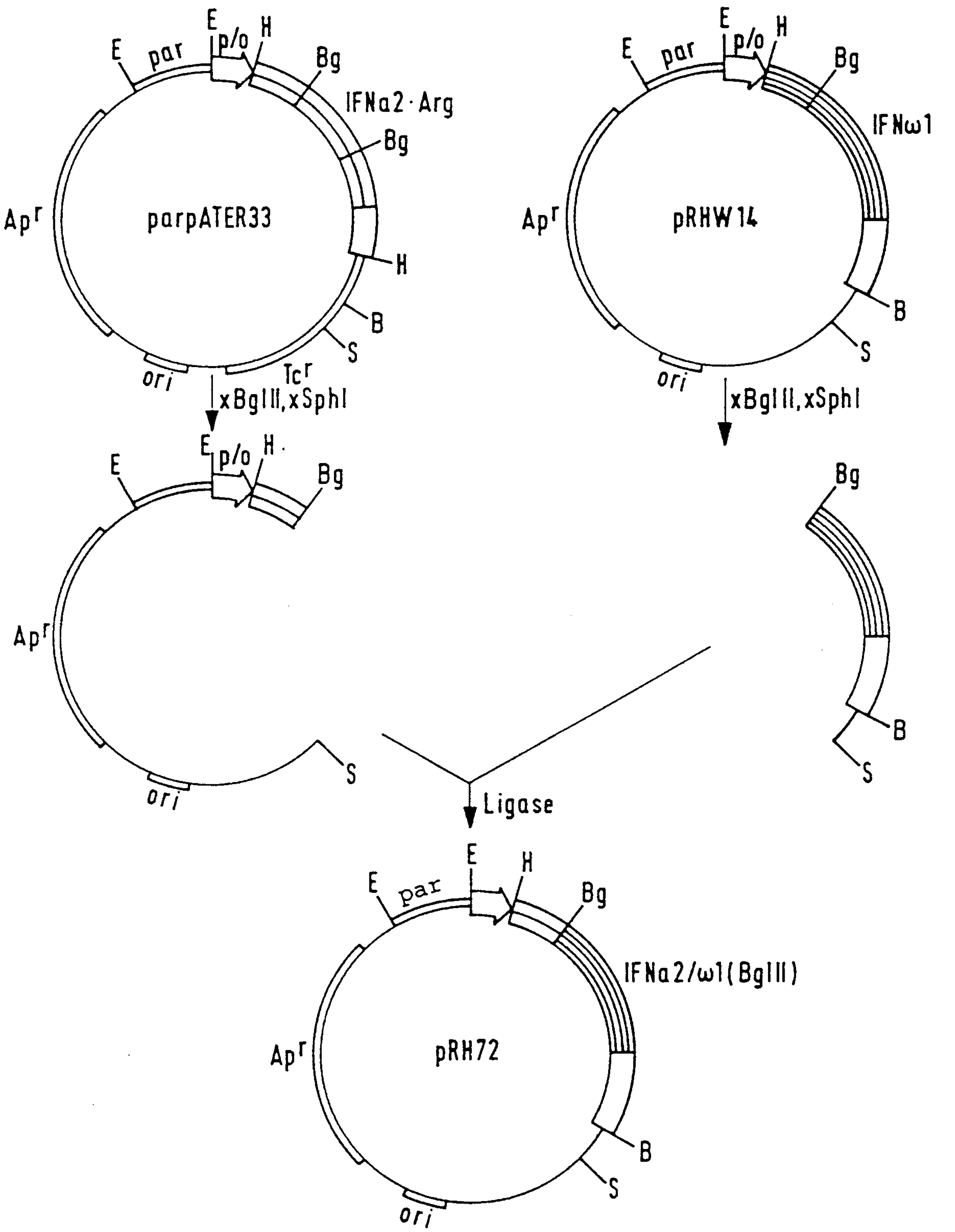
E
par
E
p/o
H
Bg
IFNa2·Arg
Bg
Apr
parpATER33
H
B
S
ori
Tcr
xBglII, xSphI
E
par
E
p/o
H
Bg
IFNω1
Apr
pRHW14
B
S
ori
xBglII, xSphI
E
E
p/o
H
Bg
Apr
S
ori
Bg
B
S
Ligase
E
par
E
H
Bg
IFNa2/ω1(BglII)
Apr
pRH72
B
S
ori

Fig. 5

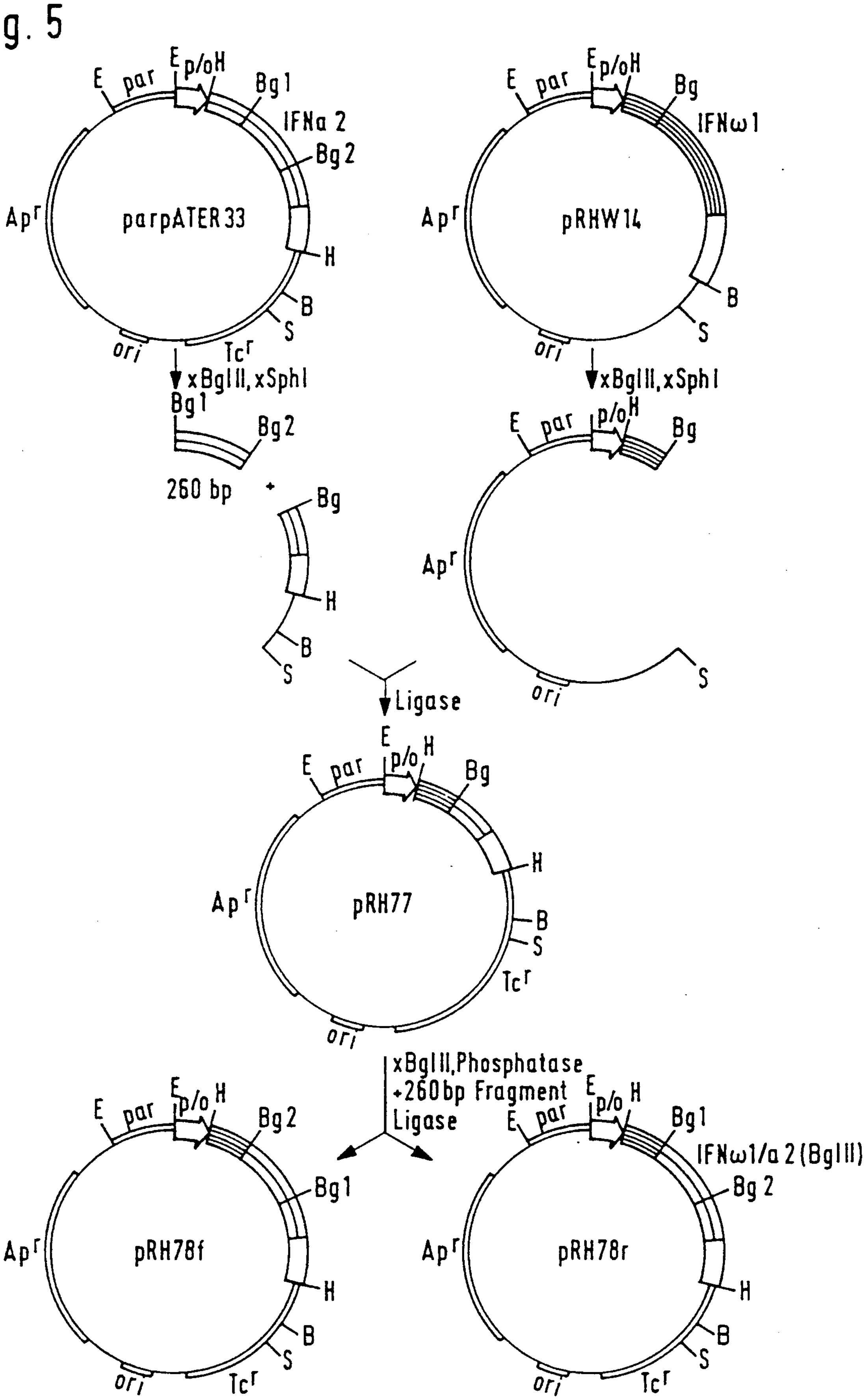
E
par
E
p/o
H
Bg1
IFNa 2
Bg2
Ap^r
parpATER33
H
B
S
ori
Tc^r
x BglII, x SphI
Bg
IFNω1
pRHW14
Bg1
Bg2
260 bp
+
Ligase
pRH77
xBglII, Phosphatase
+260bp Fragment
Ligase
pRH78f
IFNω1/a 2 (BglII)
pRH78r

Fig. 6

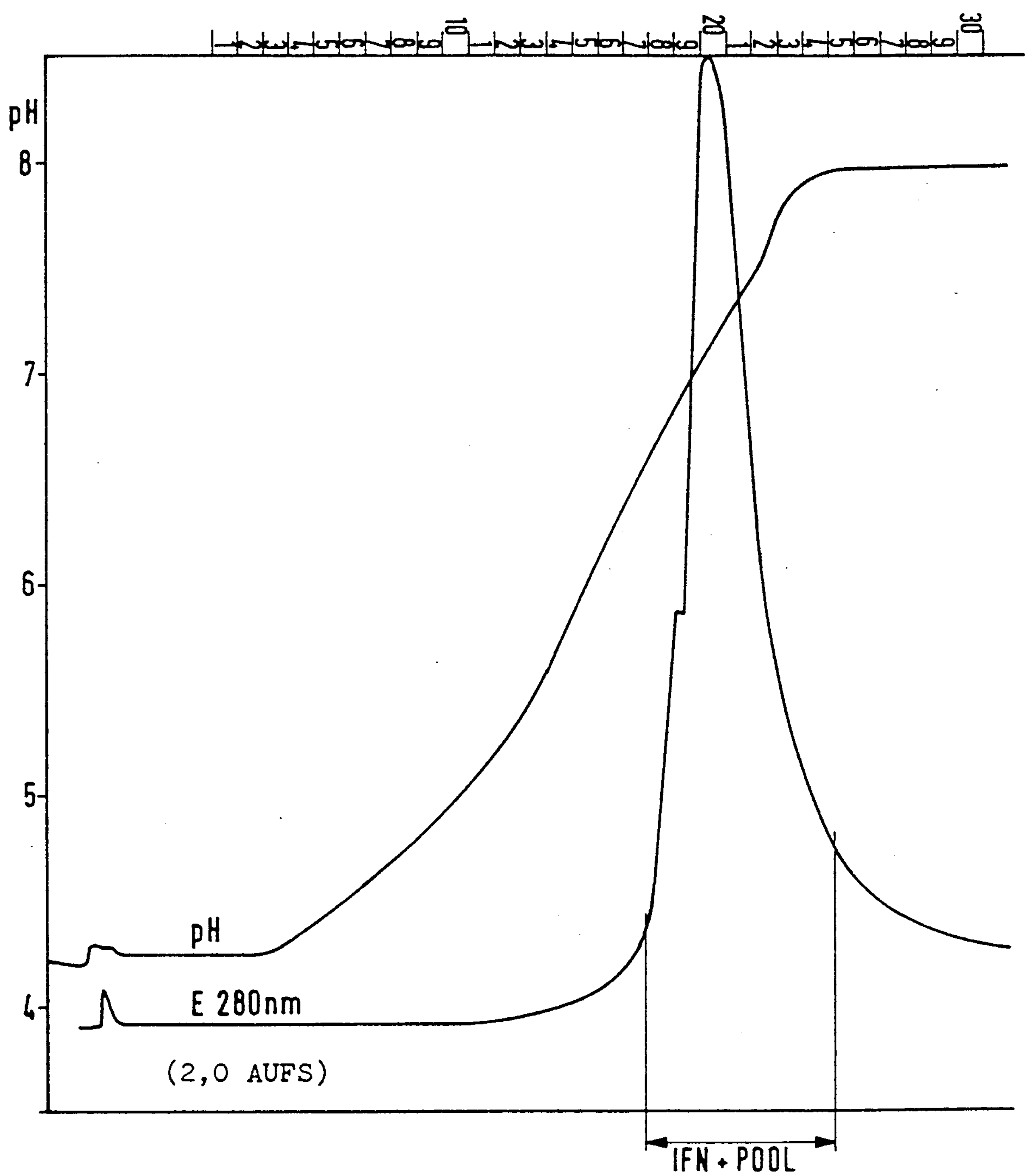

Fig. 7

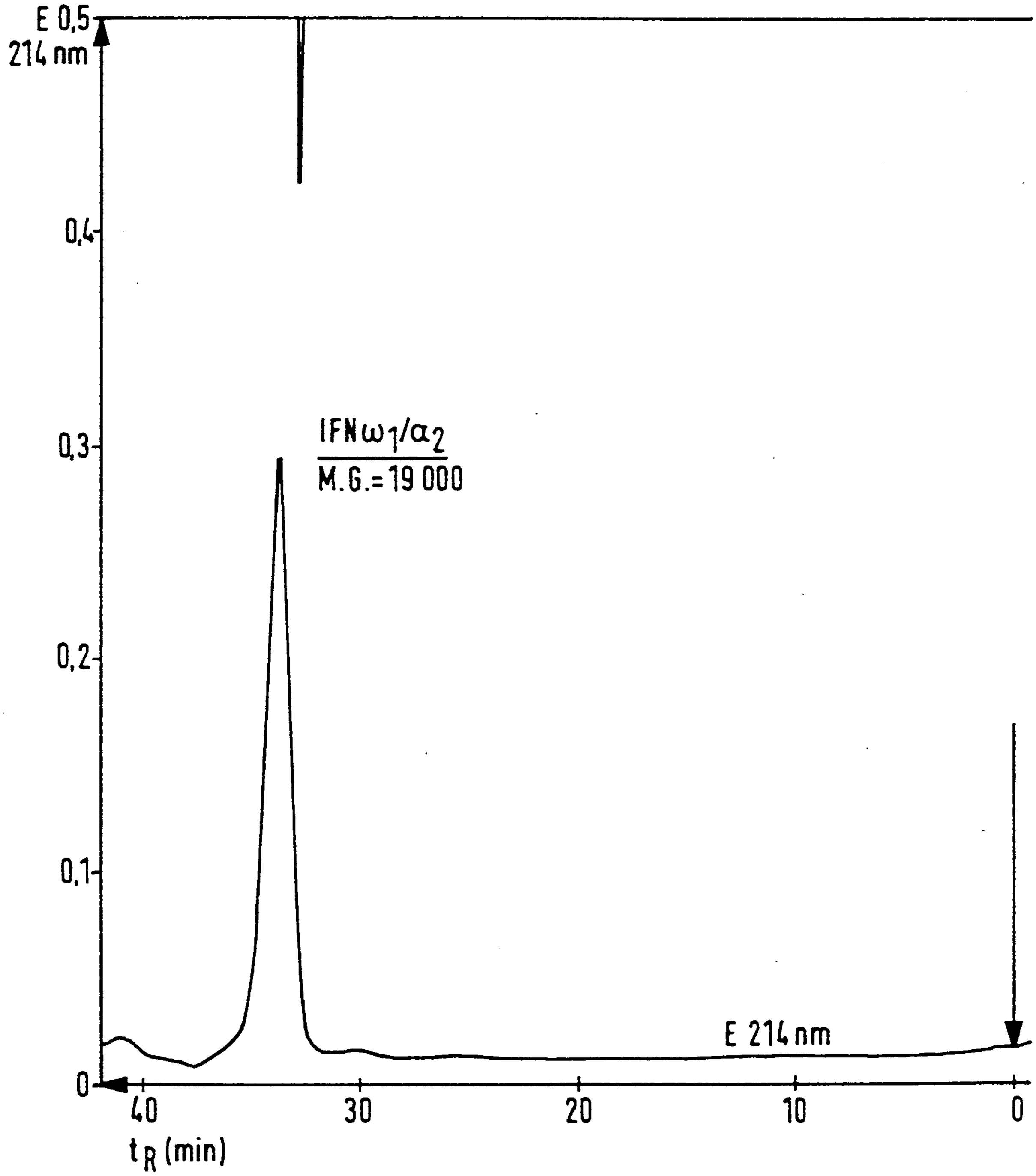
E 0,5
214 nm
0,4
0,3
0,2
0,1
0
IFNω1/α2
M.G.=19 000
E 214 nm
40
30
20
10
0
tR (min)

Fig. 8

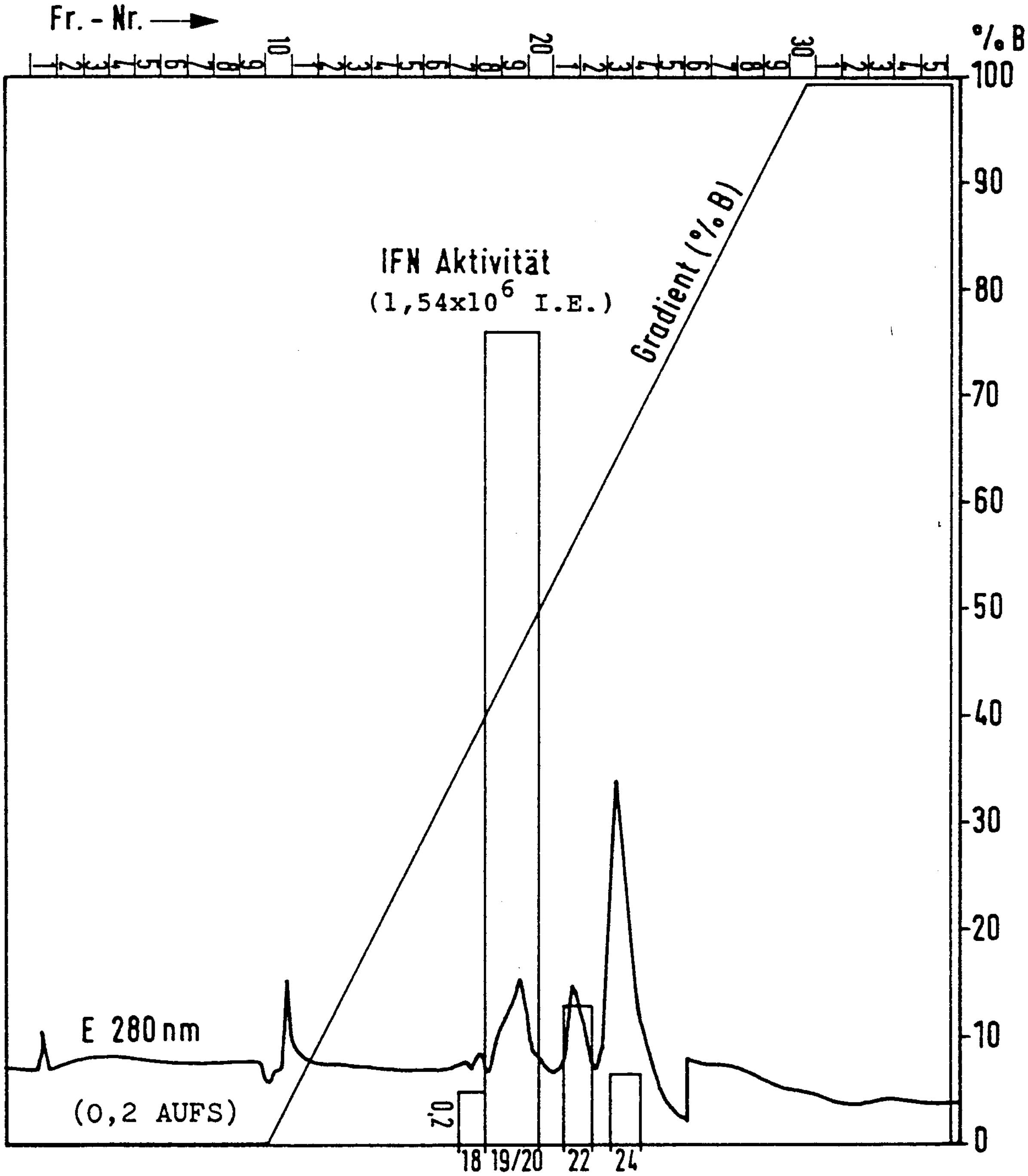
Fr. - Nr.
10
20
30
%B
100
90
80
70
60
50
40
30
20
10
0
IFN Aktivität
(1,54x10$^6$ I.E.)
Gradient (%B)
E 280 nm
(0,2 AUFS)
0,2
18
19/20
22
24

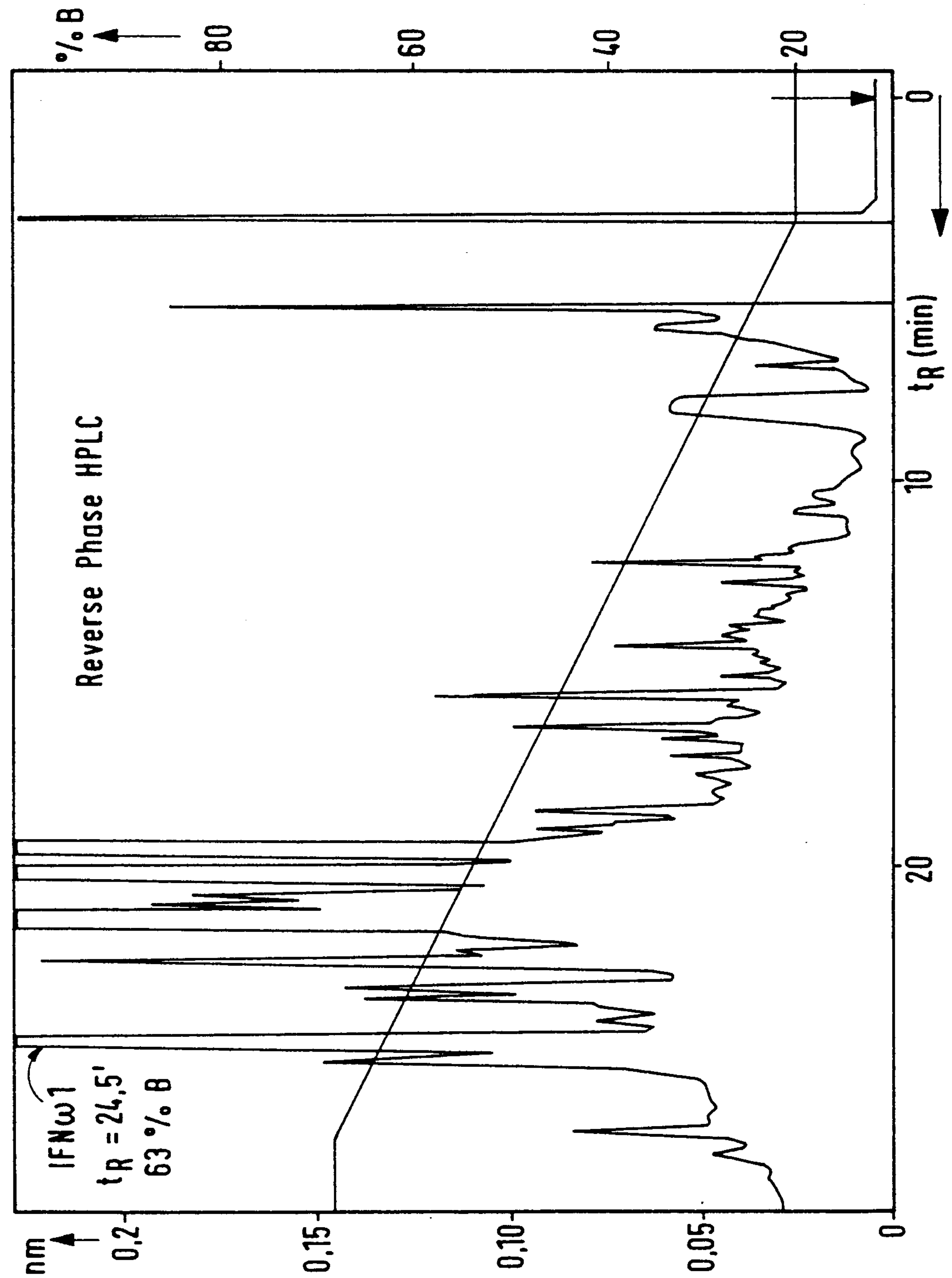
Reverse Phase HPLC
IFNω1
$t_R$ = 24,5'
63 %. B
$E_{214\,nm}$
0,2
0,15
0,10
0,05
0
%. B
80
60
40
20
0
10
20
$t_R$ (min)

Fig. 9

Europäisches Patentamt

# EUROPÄISCHER RECHERCHENBERICHT

Nummer der Anmeldung

EP 91 12 0740

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4 ) |
|---|---|---|---|
| D,A | EP-A-0 170 204 (BOEHRINGER INGELHEIM INTERNATIONAL G.M.B.H.)<br>* Seite 3, Zeile 6 - Seite 5, Zeile 9; Ansprüche *<br>* Seite 15, Zeile 11 - Zeile 23 *<br>* Seite 67, Zeile 12 - Seite 68, Zeile 23 *<br>--- | 1-9 | C12P21/08<br>C12N5/20<br>C07K3/18<br>C07K15/26<br>C07K17/00<br>//C12N15/62<br>C12N15/20 |
| A | JOURNAL OF BIOLOGICAL CHEMISTRY.<br>Bd. 257, Nr. 19, 10. Oktober 1982, BALTIMORE US<br>Seiten 11497 - 11502;<br>E. REHBERG ET AL.: 'SPECIFIC MOLECULAR ACTIVITIES OF RECOMBINANT AND HYBRID LEUKOCYTE INTERFERONS'<br>* Seite 11497, linke Spalte, Zeile 1 - Seite 11500, linke Spalte, Zeile 34 *<br>--- | 1-9 | |
| A | WO-A-8 002 229 (A/S ALFRED BENZON)<br>* Seite 3, Zeile 1 - Seite 4, Zeile 24 *<br>* Ansprüche *<br>* Zusammenfassung *<br>--- | 1-9 | |
| A | EP-A-0 051 873 (GENENTECH, INC.)<br>----- | | **RECHERCHIERTE SACHGEBIETE (Int. Cl.4 )**<br>C12P<br>C07K<br>C12N |

Der vorliegende Recherchenbericht wurde für alle Patentansprüche erstellt

| Recherchenort | Abschlußdatum der Recherche | Prüfer |
|---|---|---|
| DEN HAAG | 24 JANUAR 1992 | RYCKEBOSCH A.O. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)